# FASCICULE DE BREVET EUROPEEN

(11) **EP 0 546 930 B1**
(45) Date de publication et mention de la délivrance du brevet: **14.02.1996**
(21) Numéro de dépôt: 92403329.3
(22) Date de dépôt: 08.12.1992
(51) Int. Cl.: C07C 69/017, C07C 67/24

(54) **Procédé d'obtention d'esters d'aryle par O-déalkylation et applications**
Verfahren zur Darstellung von Arylestern durch O-Dealkylierung und Anwendungen
Process for obtaining aryle esters by o-dealkylation and applications

(30) Priorité: 13.12.1991 FR 9115548
(43) Date de publication de la demande: 16.06.1993
(73) Titulaire: SOCIETE NATIONALE DES POUDRES ET EXPLOSIFS, F-75181 Paris Cédex 04 (FR)
(72) Inventeur: Olofson, Roy, State College, Pennsylvania 16803 (US); Parrinello Lawson, Ann, Lancashire, Delaware 19810 (US); Rayle, Heather, Los Angeles, California 90034 (US)
(74) Mandataire: Phélip, Bruno

(56) Documents cités:
- WO-A-89/10913

## Description

La présente invention a pour objet un nouveau procédé d'obtention d'esters d'aryle par O-déalkylation d'alkyl aryl éther en présence de catalyseur.

Beaucoup d'opérations chimiques effectuées sur des composés porteurs de groupes hydroxy sur des cyles aromatiques imposent leur O-protection préalable pour éviter les réactions indésirables [ T.W. GREENE , Protective groups in organic synthesis, John Wiley / New York, 1981, 89-92 et HOUBEN-WEYL Methoden der organischen Chemie, Georg THIEME Verlag 1976, Band 6, Teil lC p.313 ]. En effet, les fonctions hydroxy aromatiques peuvent réagir avec de nombreux réactifs comme les électrophiles, les oxydants et même les agents d'alkylation ou d'acylation.

L'alkylation, et plus précisément la méthylation des groupes hydroxy phénoliques qui conduit aux méthyl aryl éthers, est une méthode de protection particulièrement utile en raison de la solidité des groupes alkoxy.

Malheureusement , la libération finale des fonctions hydroxyphénoliques des méthyl aryl éthers se révèle toujours difficile et requiert des conditions sévères ou des réactifs très coûteux .

Parmi les réactifs de clivage les plus communément utilisés, on peut citer :
- les acides halohydriques (HBr, HI) [ GREENE (1981), précédemment cité , p. 89 ]. Dans cette méthode, la plus ancienne , introduite par ZEISEL [ Monatsh. Chem. (1885) 6 , 989 et (1886) 7, 406 ] , on porte au reflux le substrat avec l'acide halohydrique . Les conditions réactionnelles sont tellement sévères que la plupart des substituants des noyaux aromatiques sont détruits.
- le chlorhydrate de pyridinium [R. ROYER et P. DEMERSEMAN Bull. Soc. Chim. France ( 1968 ) 2633 ] . Dan s ce cas , on porte à 200-220°C le substrat avec au moins 3 équivalents de chlorhydrate de pyridinium. Les résultats sont très dépendants de petites variations des conditions opératoires [ J.P. PEPE , Ph. D. Thesis , The Pennsylvania State University ( 1976 ) page 84 ] . De plus , plusieurs types de réactions parasites sont possibles , incluant notamment des migrations de groupes méthyle .[ J.H.P. TYMAN J. Chem . Soc. Chem. Comm. (1972) 914 ].
- les tribromure et trichlorure de bore . BBr₃ est actuellement le réactif le plus employé pour la O-déméthylation non sélective des méthyl aryl éthers [Mc OMIE et al . Tetrahedron (1968) 24, 2289 ] . Cependant , il coupe également les esters [ A.M. FELIX , J. Org. Chem. (1974) 39, 1427 ] et les acétals [ M.W. BHATT et S.V. KUSKARNI , Synthesis (1983) 249 ]. De plus , des réactions parasites interviennent en présence de fonctions cétones [ J.T. MARTZ , Ph.D. Thesis ,The Pennsylvania State University ( 1982 ) p.26] et aldéhydes [ J.M. LANSINGER et R.C. RONALD Synth. Comm. (1979 ) 9 ,341].

D'autres agents de clivage ont été proposés . Ils présentent tous à des degrés divers, de graves inconvénients , par exemple :
- Me₃SiI et Ph₂PLi qui sont des réactifs très coûteux,
- EtSNa qui est un réactif polluant , et
- NaCN/DMSO qui est un réactif très toxique.

Me , Et , Ph sont les abréviations respectives des radicaux méthyle , éthyle , phényle et DMSO est l'abréviation du diméthylsulfoxyde .

Ainsi , aucune des nombreuses méthodes de O-déméthylation décrites dans l'art antérieur ne donne des résultats pleinement satisfaisants comme illustré par J.R. HWU et S.C. TSAY [J. Org. Chem. (1990) 55 , 5987-91 ] .Ces auteurs introduisent aussi un nouveau réactif de clivage, Me₃SiSNa qui, s'il donne des résultats intéressants , est réservé au laboratoire en raison de son coût et de problèmes de pollution.

L'estérification des composés phénoliques pose aussi de nombreux problèmes . [ HOUBEN WEYL Methoden der Organischen Chemie, Georg THIEME Verlag 1985 , Band E5 Teil 1 , p.697 ].

En effet, les phénols sont sensibles à l'oxydation dans les milieux alcalins, et les esters obtenus y sont partiellement hydrolysés, ce qui rend délicat le travail en milieu basique pour réaliser la condensation d'un chlorure d'acide avec un phénol selon la réaction suivante :

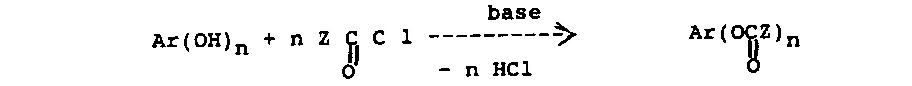

Malgré ses inconvénients, cette méthode est pratiquement la seule utilisée .

Il est à noter par ailleurs que l'obtention d'esters d'aryle par réaction d'alkyl aryl éther avec un chlorure d'acide est fort peu décrite dans la littérature . Dans une seule et très ancienne publication ( V. Prey, Chem. Ber. 75 B, n° 5 , 1942 , P.544 ) , on a fait réagir de l'anisole et du vératrole avec du chlorure de benzoyle . L'anisole ne réagit pas . Dans le cas du vératrole un mélange de 2-méthoxyphénol et de pyrocatéchol aurait été obtenu . Mais ce résultat n'a pas pu être reproduit .

WO 89/10913 décrit un procédé d'obtention d'acétates d'aryles.

On ne connaît donc pas dans l'art antérieur de méthode pour protéger la fonction hydroxy de composés phénoliques de manière efficace, stable mais facilement réversible .

La protection des fonctions hydroxy par formation d'éthers d'alkyle et d'aryle est difficilement réversible , tandis que les esters d'alkyle et d'aryle sont trop instables en milieu basique .

Selon l'invention , on a trouvé de manière surprenante que l'on pouvait dans des conditions catalytiques particulières former des esters d'aryle à partir d'alkyl aryl éthers.

La demanderesse a ainsi montré que l'on pouvait protéger de manière simple , fiable et réversible des groupements hydroxy de noyaux phénoliques.

La demanderesse a aussi établi que l'on pouvait de cette manière synthétiser des polyesters aromatiques .

La présente invention a donc pour objet un procédé d'obtention d'un ester d'aryle par O-déalkylation d'un alkyl aryl éther , caractérisé en ce qu'on fait réagir un alkyl aryl éther avec un halogénure d'acyle en présence d'un catalyseur choisi parmi les sels d'hexaalkylguanidinium et les sels de tétraalkylphosphonium.

Avantageusement , les sels de guanidinium répondent à la formule : 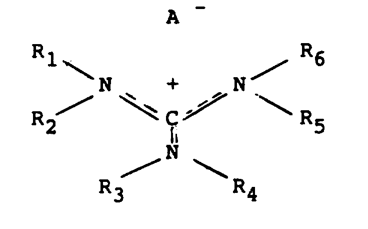 et les sels de phosphonium à la formule : 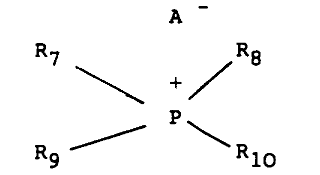 dans lesquelles R₁ à R₁₀ sont identiques ou différents et représentent des radicaux alkyles en C₁ à C₂₀ et A ⁻ représente un anion choisi dans le groupe comprenant Cl⁻, Br ⁻ I ⁻ , BF₄ ⁻ , CN⁻ , C10₄ ⁻, NO₃ ⁻, NO₂ ⁻ , OCN⁻, CH₃SO₄ ⁻, HSO₄ ⁻.

Préférentiellement les radicaux R₇ à R₁₀ contiennent de 4 à 18 atomes de carbone .

Selon une variante préférée , le procédé selon l'invention est applicable aux alkyl aryl éthers de formule Ar(OR)n dans laquelle :
R représente un groupement alkyle primaire ou secondaire en C₁ à C₆, en particulier un groupement méthyle ,
n est un nombre entier de 1 à 6 et Ar représente :
   - un radical aromatique en C₆ à C₁₄ de préférence phényle ou naphtyle , éventuellement substitué par un ou plusieurs groupements identiques ou différents, tels que des atomes d'halogène , en particulier F, Cl, Br, des groupements nitro cyano, aldéhyde des groupes alcoxy , aryloxy ou aryloxy substitué , par exemple des groupes alkyl aryloxy ou halogénoalkylaryloxy, des groupes aryl- , alkyl- ou cycloalkylcétone, des groupes aryles en particulier phényle, des groupes alkyles en C₁ à C₂₀, ramifié ou non, substitué ou non, par exemple par un groupe phényle ,
   - le groupe coumarinyle ou
   - un ensemble de plusieurs radicaux phényles, chaque radical phényle pouvant porter un ou plusieurs groupes OR , en particulier Ar(OR)n peut représenter les radicaux de formule : 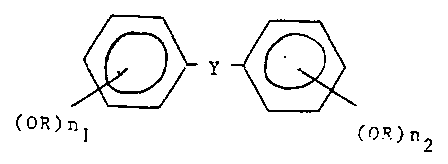 dans laquelle Y représente O, 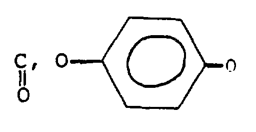 ou une liaison covalente,
et n1, n2 sont des nombres entiers de 1 à 3 identiques ou différents .

Lorsque plusieurs radicaux R sont présents dans le composé , ces radicaux peuvent être identiques ou différents .

L'halogénure d'acyle qui est mis à réagir avec l'alkyl aryl éther peut être représenté par la formule générale 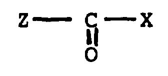 dans laquelle :
X représente un halogène , en particulier Br ou C1,
Z représente
   - un radical aromatique en C₆ à C₁₀, de préférence le radical phényle , éventuellement substitué par des radicaux choisis parmi les radicaux alkyle, les atomes d'halogène et les radicaux phénoxy ,
   - un radical aliphatique ou cycloaliphatique avec jusqu'à 20 atomes de carbone , de préférence avec de 6 à 20 atomes de carbone , saturé ou non, substitué ou non , par exemple par un groupe phényle ,
   - un radical phénoxy , lesdits radicaux pouvant également comporter ou être substitués par un autre groupe 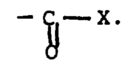
Dans ce cas on peut par réaction avec l'alkyl aryl éther Ar(OR)n avec n ≥ 2 obtenir un polyester .

Lorsque n = 1 le schéma de la réaction s'écrit comme suit :

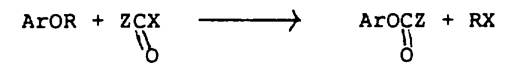

Les sels d'hexalkylguanidinium utilisés comme catalyseurs dans le procédé selon l'invention sont des composés connus qui peuvent être préparés selon les procédés décrits dans le brevet FR n° 2 585 351. De préférence les radicaux R₁ à R₆ ont de 1 à 5 atomes de carbone . Généralement l'anion est un halogénure , avantageusement un chlorure qui peut être sous forme de chlorhydrate .

Les catalyseurs particulièrement appropriés sont les chlorures d'hexabutylguanidinium (CHBG ) ou d'hexaméthylguanidinium ( CHMG ) utilisés en tant que tels ou sous forme de chlorhydrates .

Dans les sels de phosphonium les radicaux R₇ à R₁₀ comportent de préférence de 4 à 18 atomes de carbone . L'anion est celui qui est le plus facilement disponible.

Des catalyseurs qui conviennent bien sont par exemple le bromure tributyl hexadécyl phosphonium et le bromure de tétrabutylphosphonium.

La réaction de O-déalkylation des alkyl aryl éthers réalisée selon l'invention, permet d'éviter l'emploi de réactifs coûteux ou polluants et empêche la modification ou l'élimination des autres substituants du noyau aromatique.

L'invention peut s'appliquer à la déprotection des fonctions -OH aromatiques temporairement protégées sous forme d'alkyl aryl éthers et, plus précisément de méthyl aryl éthers.

Les esters d'aryles obtenus au moyen du procédé de l'invention peuvent être hydrolysés facilement en milieu basique , en particulier par la méthode classique décrite dans HOUBEN WEYL ( Methoden der Organischen Chemie , Georg THIEME, Verlag , 1976 , Band 6 , Teil Ic , 436-451 ) pour obtenir les phénols .

Pour cette application Z représente de préférence le radical phényle .

Comme exposé par Greene ( précédemment cité ) , les groupes hydroxyphénoliques sont présents dans de nombreux composés d'intérêt biologique tels que la tyrosine , la thyroxine , l'estrone , la codéine , la terramycine, l'adrénaline , la naloxone .

Une étape de déméthylation d'une fonction méthyl aryl éther peut intervenir dans la synthèse de nombreux autres produits comme par exemple des pesticides . Ainsi le brevet DE 3 832 656 ( cf exemple 1 ) utilise dans la préparation d'acaricides la déméthylation du composé

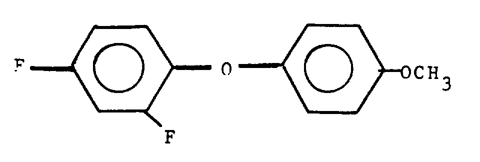

Une deuxième application de l'invention est la synthèse d'esters ou de polyesters aromatiques . Un grand nombre de benzoates d'aryle ont un grand intérêt dans l'industrie, par exemple :
- pour la fabrication de cristaux liquides [ EP 99.488 ]
- pour la reprographie sur papier thermosensible [US 4.500.896]
- pour les photosensibilisateurs [ DE 3.007.797 ]
- pour la stabilisation des polymères [ US 4.110.301],
- pour des absorbants anti-UV [ DE 3.417.782 ].

Le procédé selon l'invention peut également servir pour la préparation de polyesters possédant de très bonnes propriétés thermiques [ J. Macromol. Sci. Chem. (1985) A 22 (5-7) p.561-577 ] .

De manière générale on peut mettre en oeuvre l'invention dans les conditions réactionnelles suivantes . On emploie de manière avantageuse de 0,01 à 0,1 équivalent de catalyseur par mole de fonction -OR à cliver . Les catalyseurs qui ont été exposés à l'humidité doivent être séchés avant leur utilisation .

En outre , la température de réaction doit être de manière avantageuse comprise entre 150°C et 220°C et de préférence entre 180°C et 200°C.

On emploie généralement de 1,0 à 3,0 équivalent de fonction halogénure d'acyle par fonction alkyléther à cliver et, de préférence 1,0 à 1,5 équivalents quand n = 1.

On préfère , lorsque cela est possible , opérer sans solvant . Dans le cas d'alkyl aryl éthers ayant des points de fusion élevés (supérieurs à 150°C) ou lorsque l'on veut préparer des polymères , on peut utiliser un solvant inerte préférentiellement ayant un point d'ébullition supérieur à 150°C , tel que l'o-dichlorobenzène, le 2,4-dichlorotoluène, le 1,2,4-trichlorobenzène.

La durée de la réaction est généralement comprise entre 8 et 24 heures.

La présente invention est illustrée sans pour autant être limitée par les exemples suivants .

Dans ces exemples, les catalyseurs ont été séchés avant emploi à 100°C sous vide pendant plusieurs heures pour le chlorure d'hexabutylguanidinium ( CHBG ) ou le chlorure d'hexaméthylguanidinium ( CHMG ) ou à 20°C pendant 20 min sous vide pour le CHBG, HCl ou le CHMG, HCl.

### EXEMPLE 1:

### O-Déméthylation du p-anisonitrile en benzoate de 4-cyanophényle.

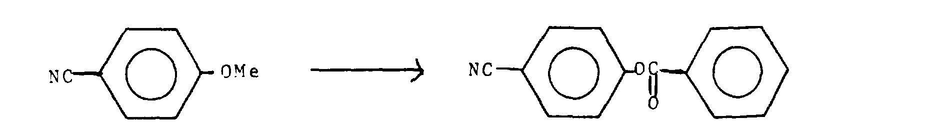

### A ) Catalyse par le CHBG

On place sous agitation à 185°C pendant 13 heures une solution de 0,19 g (0,45 mmol ; 0,04 eq) de CHBG, 1,50g (11,3 mmol ) d'anisonitrile et 1,60 g (11,4 mmol ) de chlorure de benzoyle dans 5 ml d'o-dichlorobenzène (O-DCB).

Après distillation du milieu réactionnel sous pression réduite (80°C/ 1 mm Hg ) , on dissout le résidu solide jaune dans 50 ml de CHCl₃ et agite 20 min avec 1,5 g de silice . La silice ayant adsorbé le catalyseur est séparé par filtration . Après élimination du solvant par évaporation sous pression réduite , le produit attendu est purifié par recristallisation dans un mélange CCl₄/hexane. On obtient 3,26 g d'aiguilles blanches soit un rendement de 96 % .

Les caractéristiques du produit sont les suivantes :
Point de fusion (P.F.) : 93-94,5°C
IR(CHCl₃) ν : 2180 (m), 1740 cm⁻¹ (s)
RMN ¹H (CDCl₃) δ : 8,21 ( d de d, 2H, J = 7 - 1 Hz); 7,8-7,65(m,3H);
7,6 - 7,5 ( m, 2H) ; 7,35 ( d de d, 2H, J = 8,5 - 2 Hz).

### B ) Catalyse par le bromure de tributylhexadécylphosphonium.

On agite un mélange de 1,73 g ( 13 mmol) d'anisonitrile avec 2,07 g ( 14,7 mmol) de chlorure de benzoyle et 0,33 g (0,65 mmol, 0,05 éq ) de bromure de tributylhexadécylphosphonium à 185°C pendant 16 heures . Le solide marron obtenu est ensuite agité dans 75 ml de CH₂Cl₂ avec 3 g de silice . On filtre , on concentre le filtrat et on obtient un solide qui est recristallisé . 2,74 g du produit attendu sont isolés sous forme d'aiguilles blanches (rendement 94 % ) P.F. : 93-95°C.

### C ) Essai comparatif sans catalyseur :

Pour démontrer le rôle du catalyseur , on a répété l'essai A avec les mêmes conditions , mais sans ajouter de catalyseur :
Même après chauffage à 185°C pendant 60 h , le mélange obtenu ne contient aucune trace de benzoate de 4-cyanophényle. On recueille les produits de départ et une faible quantité (< 9%) d'une impureté non identifiée .

### EXEMPLE 2 :

### Bis (O-déméthylation) de la 4,4'-diméthoxybenzophénone.

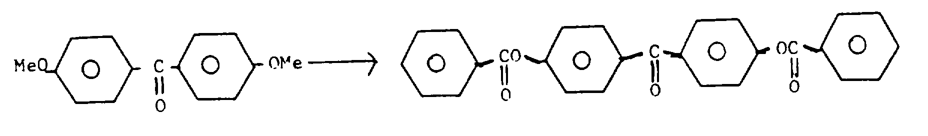

On place sous agitation à 185°C pendant 20 h , un mélange de 0,12 g ( 0,26 mmol) de CHBG, HCl, 1,04 g ( 4,29 mmol) de 4,4'-diméthoxybenzophénone et 1,85 g (13,2 mmol) de chlorure de benzoyle.

Après élimination de l'excès de chlorure de benzoyle par distillation sous pression réduite, le résidu est traité avec de la silice comme indiqué à l'exemple 1.

Le produit recueilli est lavé à l'hexane et séché sous vide . On obtient 1,73 g d'une poudre blanche ( rendement 98%) qui présente les caractéristiques suivantes :
P.F. : 183,5-184,5°C. IR(CHCl3), 1740 (s), 1240 (s) , 1140 cm⁻¹(m),
RMN¹ H δ: 8,3 - 8,2 (m, 4H); 8,0 - 7,9 ( m, 4H); 7,7-7,65 (m,2H); 7,6-7,5 (m,4H), 7,4-7,35 (m,4H).

### EXEMPLE 3

### O-Déméthylation du 2,4-diméthylanisole en benzoate de 2,4-diméthylphényl.

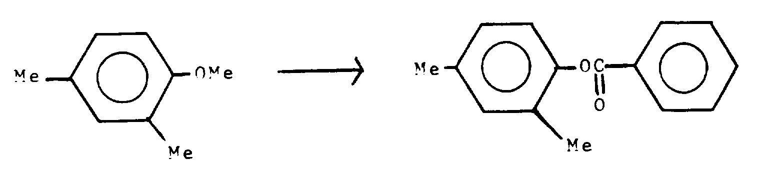

### A ) Catalyse par le CHBG

On place sous agitation à 185°C pendant 19 heures , un mélange de 0,21 g ( 0,44 mmol) de CHBG,HCl, 1,54 g (11,3 mmol) de 2,4-diméthylanisole et 1,78 g (12,7 mmol) de chlorure de benzoyle . On purifie directement le mélange brut obtenu par chromatographie sur couches minces ( CH₂Cl₂/ hexane , V/V 22/75 R_{f} : 0,28). On recueille ainsi 2,48 g (rendement 97%) d'un liquide clair visqueux qui , par refroidissement à -10°C, donne des cristaux blancs (P.F. : 37-39°C) présentant les caractéristiques suivantes :
IR(CCl₄) 1740 (s), 1240 (s), 1170 cm⁻¹ (s) RMN ¹H (CDCl3) δ 8,35-8,25 (m, 2H); 7,7-7,65 (m,
1H); 7,6-7,5 (m,2H); 7,15-7,0 (m, 3H); 2,4(s, 3H); 2,25 (s, 3H).

### B ) Catalyse par le bromure de tétrabutylphosphonium.

On agite à 185°C pendant 20 heures un mélange de 0,18 g (0,54 mmol , 0,10 eq.) de bromure de tétrabutylphosphonium , de 0,76 g ( 5,58 mmol) de 2,4-diméthylanisole et de 0,87 g ( 6,19 mmol) de chlorure de benzoyle . L'huile jaune brute obtenue est purifiée par chromatographie ( 24/76 CH₂Cl₂/hexane ) . On obtient 1,13 g du produit attendu ( rendement 89 % ) .

### C) Essai comparatif sans catalyseur.

Pour démontrer le rôle du catalyseur, on a répété l'essai A sans ajouter de catalyseur :
Même après chauffage à 185°C pendant 60 heures, le mélange obtenu ne contient aucune trace de 2,4-diméthylphénylbenzoate .

### EXEMPLE 4:

### O-déméthylation du p.anisonitrile en 2-éthyl hexanoate de 4-cyanophényle.

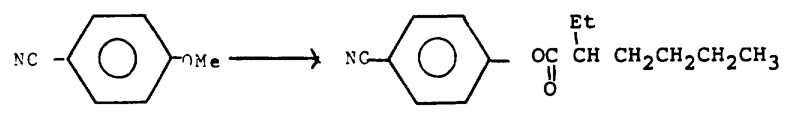

On place sous agitation à 185°C pendant 13 heures une solution de 0,18 g (0,41 mmol) de CHBG, 1,32 g (9,88 mmol) de p-anisonitrile et 1,67 g ( 10,1 mmol ) de chlorure d'éthyl-2 hexanoyle dans 4 ml d'o-dichlorobenzène . Après élimination du solvant par distillation sous pression réduite, on purifie le produit brut obtenu par chromatographie sur colonne (éthylacétate 6/94, hexane ; R_{f} = 0,37 ).

On obtient ainsi 2,36 g (rendement 98%) du produit attendu sous forme d'une huile légèrement jaune présentant les caractéristiques suivantes :
IR(CCl₄) 2180 (m) 1765 cm⁻¹ (s)
RMN ¹H (CDCl₃) δ 7,7-7,65 (m,2H) ; 7,25-7,15 ( m, 2H) ;
2,6-2,45 ( m,1H) ; 1,85 - 1,5 (m , 4H) ; 1,4- 1,3 (m,4H), 1,05- 0,9 (m,6H).

### EXEMPLE 5:

### O-débutylation du n-butylphényléther en benzoate de phényle.

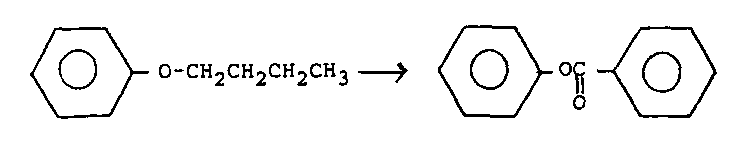

On place sous agitation à 185°C pendant 40 heures, un mélange de 1,04 g ( 6,92 mmol) de n-butylphényléther , 0,26 g ( 0,56 mmol) de CHBG, HCl et 1,21 g (8,61 mmol ) de chlorure de benzoyle. Le mélange brut obtenu est purifié par chromatographie flash (35/65, CH₂Cl₂ / hexane , R_{f} : 0,25).

On obtient ainsi 1,29 g (rendement 94%) du produit attendu , sous forme d'un produit cristallin blanc (P.F. 68,5 - 70°C) présentant les caractéristiques suivantes : IR(CDCl₃) : 1730 (s), 1245 (s) 1180 (s) cm⁻¹ RMN ¹H (CDCl₃) δ : 8,25-8,15 (m, 2H); 7,65-7,6 (m, 1H); 7,55-7,35 (m,4H) 7,3-7,15 (m,3H).

### EXEMPLE 6:

### O-déméthylation du p.anisonitrile en palmitoate de 4-cyanophényle.

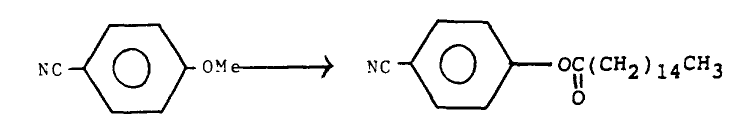

On place sous agitation à 185°C pendant 12 heures, un mélange de 0,23 g ( 0,49 mmol) de CHBG, HCl, 1,65 g ( 12,4 mmol) de p.anisonitrile et 4,10 g (14,9 mmol) de chlorure de palmitoyle . Après traitement avec de la silice comme indiqué à l'exemple 1 , le résidu brun obtenu est recristallisé deux fois dans le n-hexane.

On obtient ainsi 4,13 g (rendement 93%) du produit attendu sous forme d'aiguilles blanches (P.F. 71-74°C) présentant les caractéristiques suivantes :
IR(CDCl₃) : 2190 (m) , 1765 (s) , 1190 (s), 1145 cm⁻¹
RMN ¹H (CDCl3) δ 7,7-7,65 (m, 2H) ; 7,25-7,2 (m,2H); 2,58
(t,2H, J = 7,5 H_{z}); 1,85-1,7 (m,2H); 1,45-1,2(m, 24 H) ; 0,88 (t, 3H, J = 6,5 H_{Z}).

### EXEMPLE 7 :

### O-déméthylation du p.anisonitrile en trans-cinnamate de 4-cyanophényle.

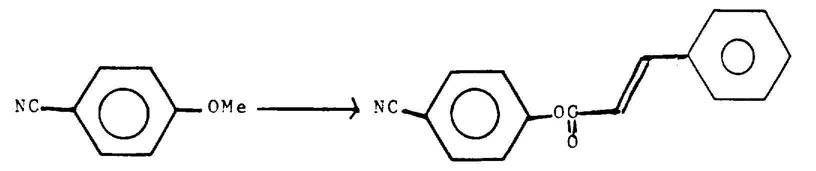

On place sous agitation à 185°C pendant 12 heures un mélange de 0,14 g ( 0,29 mmol) de CHBG, HCl, 0,97 g ( 7,29 mmol) de p.anisonitrile et 1,34g (8,04 mmol) de chlorure de trans-cinnamoyle .

On élimine les produits volatils par distillation sous pression réduite et on traite avec de la silice comme indiqué à l'exemple 1.

Le solide obtenu est débarrassé du chlorure de cinnamoyle résiduel par lavage à l'hexane et recristallisé dans le mélange hexane/CCl₄.

On obtient ainsi 1,68 g ( rendement 93%) d'une poudre blanche (P.F. 104,5-106°C) présentant les caractéristiques suivantes :
IR (CDCl₃): 2185 (m) , 1730 (s), 1630 (s) , 1190 (s),
1110 cm⁻¹ (s),
RMN ¹H (CDCl₃) : δ 7,91 ( d, 1H J = 16 H_{z}); 7,8-7,7 ( m,2H);
7,65 - 7,55 ( m,2H) ; 7,5-7,4 ( m, 3H) ; 7,35-7,3 ( m, 2H) ,
6,62 ( d, 1H J = 16 H_{Z}).

### EXEMPLE 8 :

### O-déméthylation de l'o-chloroanisole en benzoate de 2-chlorophényle.

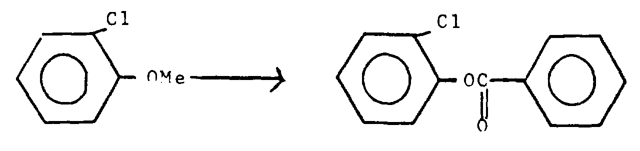

On place sous agitation à 185°C pendant 20 heures un mélange hétérogène de 0,12 g ( 0,57 mmol) de CHMG, HCl, 2,08 g (14,6 mmol) de 2-chloroanisole , 2,10 g ( 14,9 mmol) de chlorure de benzoyle et 3 ml d'o-dichlorobenzène . Après purification du produit par chromatographie sur colonne (20/80, CH₂Cl₂/hexane, R_{f} : 0,19) , on obtient 2,49 g (rendement 73%) du composé attendu sous forme d'un liquide légèrement jaune présentant les caractéristiques suivantes : IR (CCl₄) : 1750 (s) , 1220 (s), 1195 (s) , 1030 cm⁻¹ (s) RMN ¹H (CDCl₃): δ 8,3-8,2 ( m, 2H) ; 7,75-7,6 (m, 1H); 7,6-7,5 (m, 3H); 7,4-7,2 ( m, 3H).

### EXEMPLE 9:

### O-déméthylation du p.anisonitrile en benzoate de 4-cyanophényle en utilisant le bromure de benzoyle.

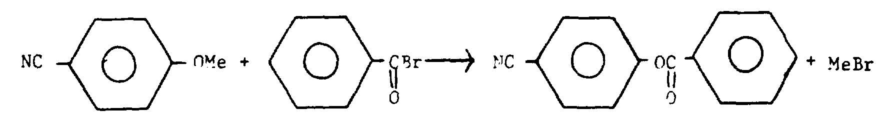

On place sous agitation à 185°C un mélange de 0,10 g (0,24 mmol) de CHBG, 0,79 g ( 5,93 mmol) de p.anisonitrile et 1,28 g ( 6,92 mmol) de bromure de benzoyle jusqu'à ce que l'analyse RMN ¹H indique la fin de la réaction ( 4 heures).

Le produit brut obtenu est dissous dans 75 ml de CHCl₃, traité avec 1,5 g de silice , filtré et concentré. Le solide obtenu est redissous dans CHCl₃, et lavé avec 20 ml d'une solution aqueuse saturée en Na₂CO₃.

Après élimination du solvant par distillation sous pression réduite et recristallisation dans le mélange hexane/CCl₄ on obtient 1,25 g ( rendement 95%) du produit attendu dont les caractéristiques sont les mêmes que celles indiquées à l'exemple 1.

### EXEMPLE 10 :

### O-déméthylation du 2-méthoxynaphtalène en benzoate de 2-naphtyle.

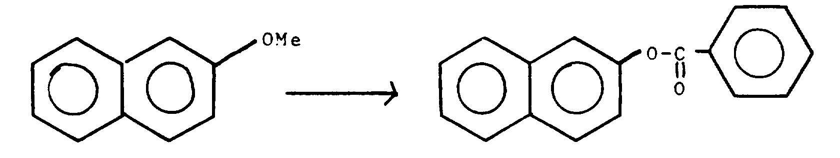

On place sous agitation à 185°C pendant 20 heures un mélange de 0,17 g (0,36 mmol) de CHBG,HCl, 1,47 g ( 9,29 mmol) de 2-méthoxynaphtalène et 1,45 g (10,3 mmol) de chlorure de benzoyle . Le solide obtenu est dissous dans 75 ml de CH₂Cl₂ et la solution est traitée avec 3 g de silice.

Après élimination du solvant par évaporation et purification par chromatographie flash (26/74, CH₂Cl₂ dans l'hexane R_{f} : 0,19) on obtient 2,15 g ( rendement : 93%) du produit attendu ( P.F. 106,5 - 107,5°C ) présentant les caractéristiques suivantes :
IR ( CHCl₃) : 1735 (s) , 1255 cm⁻¹ (s)
RMN ¹H (CDCl₃) : δ 8,35-8,25 (m,2H); 8,0-7,85 (m,3H); 7,73 (d,
1H, J = 2,2 H_{z}); 7,7-7,65 ( m, 1H); 7,6-7,5(m,4H), 7,40 ( d de d, 1H, J = 8,9 - 2,3 H_{z}).

### EXEMPLE 11:

### O-déméthylation du 4-méthoxyphényl-4'-trifluorométhylphényléther en 4-benzoyloxyphényl 4'- trifluorométhylphényléther.

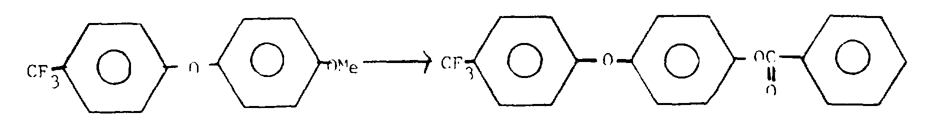

On place sous agitation à 185°C pendant 18 heures un mélange de 0,22 g ( 0,47 mmol) de CHBG, HCl, 2,53 g ( 9,43 mmol) de 4-méthoxyphényl 4'-trifluorométhylphényléther et 1,44 g (10,2 mmol) de chlorure de benzoyle.

Après élimination de l'excès de chlorure de benzoyle et traitement avec de la silice comme indiqué à l'exemple 1 on obtient 3,38 g d'un solide blanc de pureté supérieure à 99%.

La recristallisation dans l'hexane donne 3,14 g (rendement : 93%) du produit attendu ( P.F. : 95-96°C ) et qui présente les caractéristiques suivantes :
I.R. : (CCl₄) 1735 (s) , 1485 (s) , 1310 (s), 1180 cm⁻¹ (s)
RMN ¹H (CDCl₃): δ 8,3-8,2 ( m,2H); 7,7-7,45 (m,5H); 7,3-7,2
(m,2H); 7,15-7,0 ( m,4H).

### EXEMPLE 12.

### O-déméthylation du 4-méthoxystilbène en 4-benzoyloxystilbène.

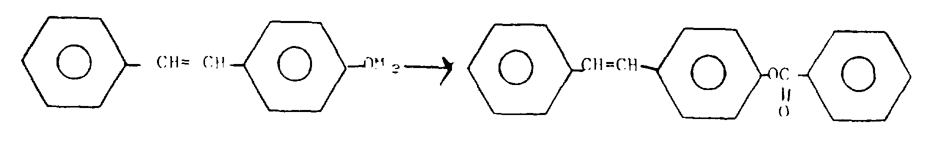

On place sous agitation à 185°C pendant 15 heures un mélange de 0,17 g ( 0,36 mmol) de CHBG, HCl, 1,49 g ( 7,09 mmol) de 4-méthoxystilbène et 1,09 g (7,75 mmol) de chlorure de benzoyle . Le solide rouge-orangé obtenu est trituré avec CCl₄ et filtré . Après recristallisation dans le toluène , on recueille 1,93 g ( rendement 91%) du produit attendu ( P.F. : 195-196°C ) et qui présente les caractéristiques suivantes : IR ( pastille KBr) :1730 (s), 1590 (w), 1490 (m), 1250 (s), 1180 cm⁻¹ (s) RMN ¹H (CDCl₃): δ 8,3-8,2 (m) (m, 2H) ; 7,7-7,5(m,8H); 7,45-7,2 (m,6H); 7,12 (s, 2H).

### EXEMPLES 13 à 28.

### Monodéméthylations de méthylaryléthers réalisées selon le mode opératoire de l'exemple 1.

La réaction est effectuée :
- à 185°C sauf dans l'exemple 22 dans lequel la réaction est effectuée à 160°C,
- avec ou sans solvant ,
- en présence de 0,04 eq de catalyseur.

Les produits obtenus ainsi que les rendements sont indiqués dans le tableau I ci-après pour chacun des éthers.

### EXEMPLES 29 à 45:

### Poly(O-déméthylations) de méthylaryléthers réalisées selon le mode opératoire de l'exemple 2.

La réaction est effectuée :
- à 185°C
- avec ou sans solvant.

Le tableau II ci-après indique les produits obtenus ainsi que les rendements des réactions.

### EXEMPLES 46 à 53.

### Hydrolyse de benzoates obtenus dans les exemples précédents en vue de libérer la ou les fonctions hydroxy.

Les opérations sont réalisées au reflux dans le dioxanne par traitement avec une solution aqueuse de soude 3,8 M dans les conditions suivantes :
- dioxanne : 100 ml pour 20-25 g d'ester
- NaOH: 2 à 4 eq par fonction ester à hydrolyser
- reflux du mélange : environ 1 h .

Après acidification par de l'acide chlorhydrique concentré, on ajoute 1000 ml d'éther , on lave la phase aqueuse avec 500 ml d'éther, puis les phases éthérées combinées avec 250 ml d'eau et on sèche sur MgSO₄.

Après évaporation de l'éther sous pression réduite, on triture le solide avec 300 ml de chloroforme . Le produit insoluble obtenu est purifié sur colonne (silica gel pour chromatographie flash, 40µm), éluant 30/70, acétate d'éthyle/hexane.

Les produits obtenus et les rendements des réactions sont mentionnés dans le tableau III ci-après.

### EXEMPLE 54.

### Préparation d'un polymère par réaction de la 4,4'-diméthoxybenzophénone avec le chlorure d'azélaoyle.

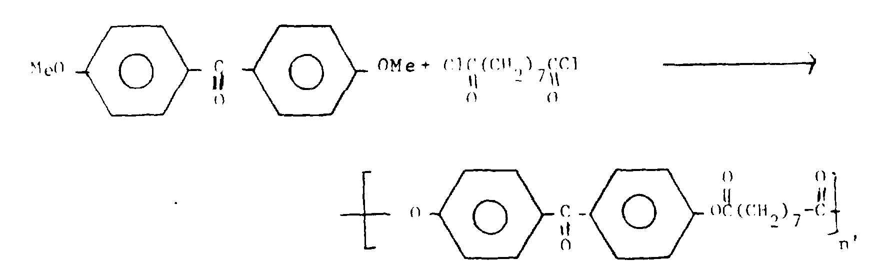

On place sous agitation à 185°C pendant 60 heures un mélange de 0,33 g ( 0,69 mmol) de CHBG,HCl, 2,1 g ( 8,60 mmol) de 4,4' -diméthoxybenzophénone, 1,935 g ( 8,60 mmol) de chlorure d'azélaoyle et 5 ml d'o-dichlorobenzène.

Le mélange est ensuite traité comme indiqué à l'exemple 1 ,( dissolution dans 100 ml de CHCl3) et le polymère attendu est recueilli sous forme d'une poudre par précipitation à l'hexane .

### Masse moléculaire : environ 39000.

### EXEMPLE 55.

### 0-Déméthylation du 1,2-diméthoxybenzène en 1,2-dibenzoyloxybenzène.

Un mélange de 0,15 g ( 0,03 éq, 0,35 mmol) de CHBG, de 1,45 g ( 10,5 mmol) de vératrole ( 1,2-diméthoxybenzène) et de 4,5 g ( 32 mmol) de chlorure de benzoyle a été agité à 185°C pendant 46 heures . Les réactifs en excès ont été distillés sous vide et le catalyseur a été éliminé par absorption sur silice à partir d'une solution dans CH₂Cl₂. On obtient après évaporation du solvant 3,32 g ( rendement brut 99%) d'un solide marron clair . On isole le produit pur sous forme d'un solide blanc par cristallisation dans de l'éthanol. Ses caractéristiques sont les suivantes :
P.F. 86-87°C.
RMN ¹H (CDCl₃ ) δ 8,05 - 8,1 ( d , 4H) , 7,5 - 7,6 ( m, 2H), 7,3-7,45 ( m,8H).
IR (CCl₄) C=O raie à 1750 cm⁻¹.

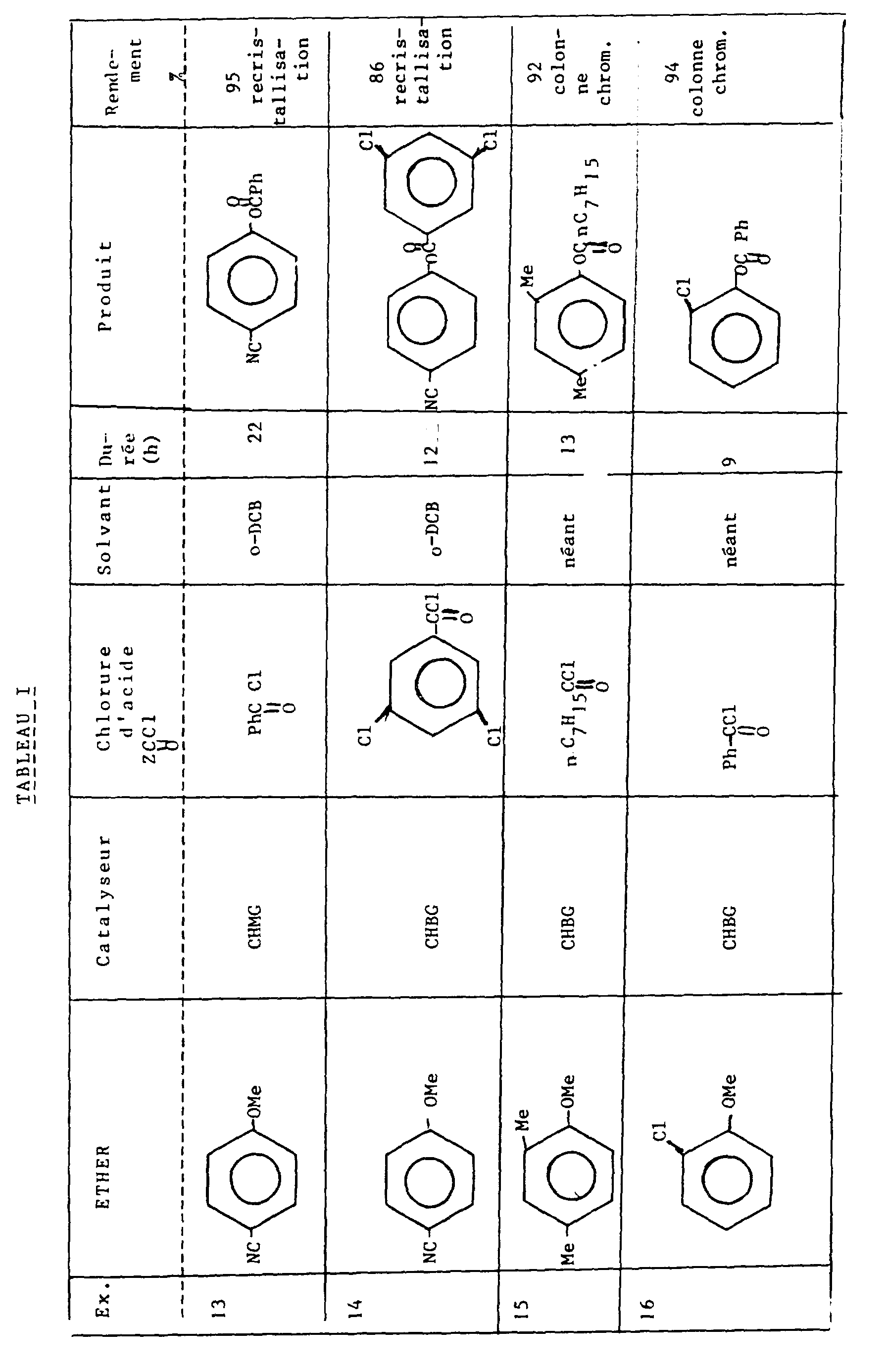

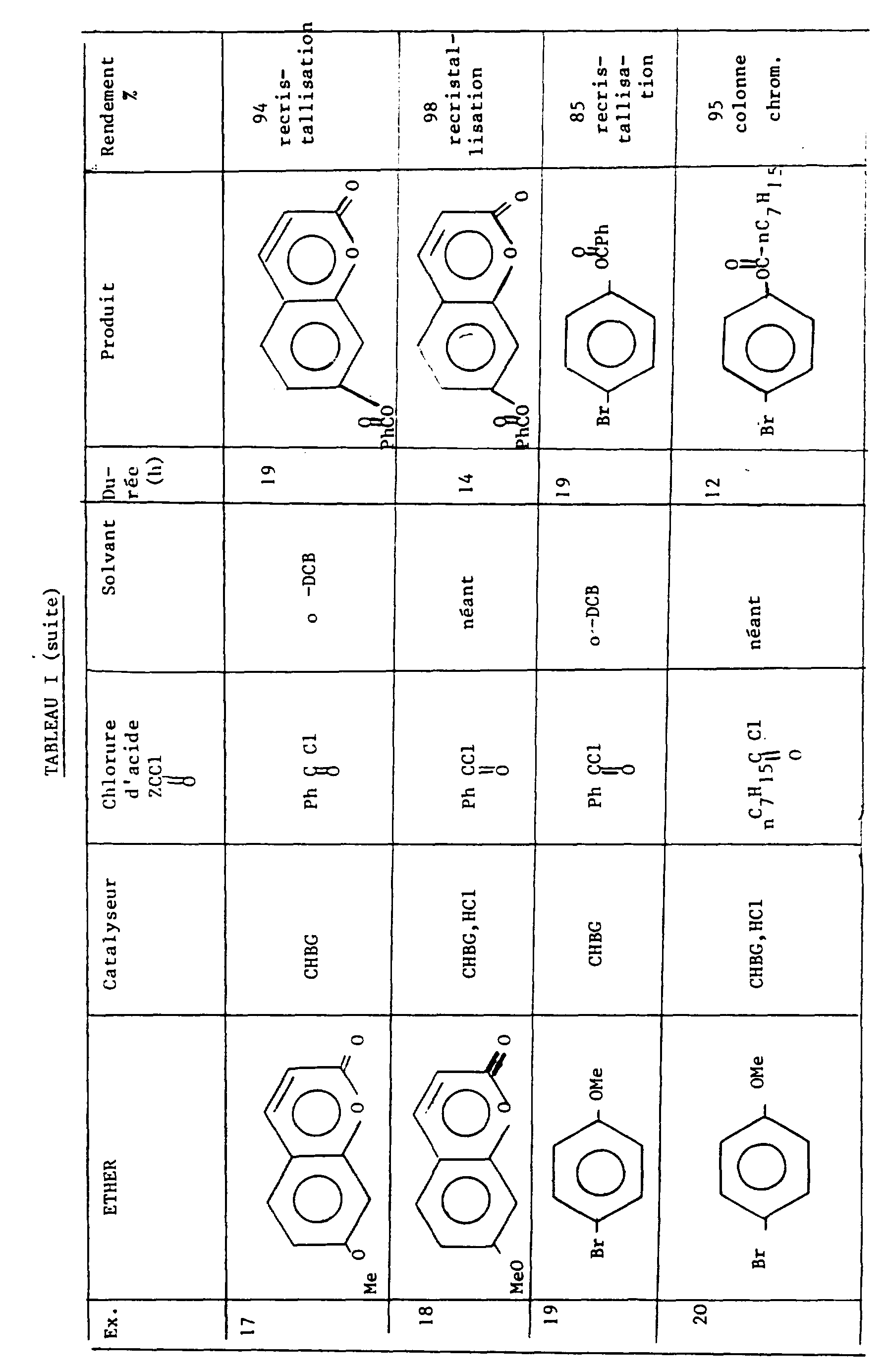

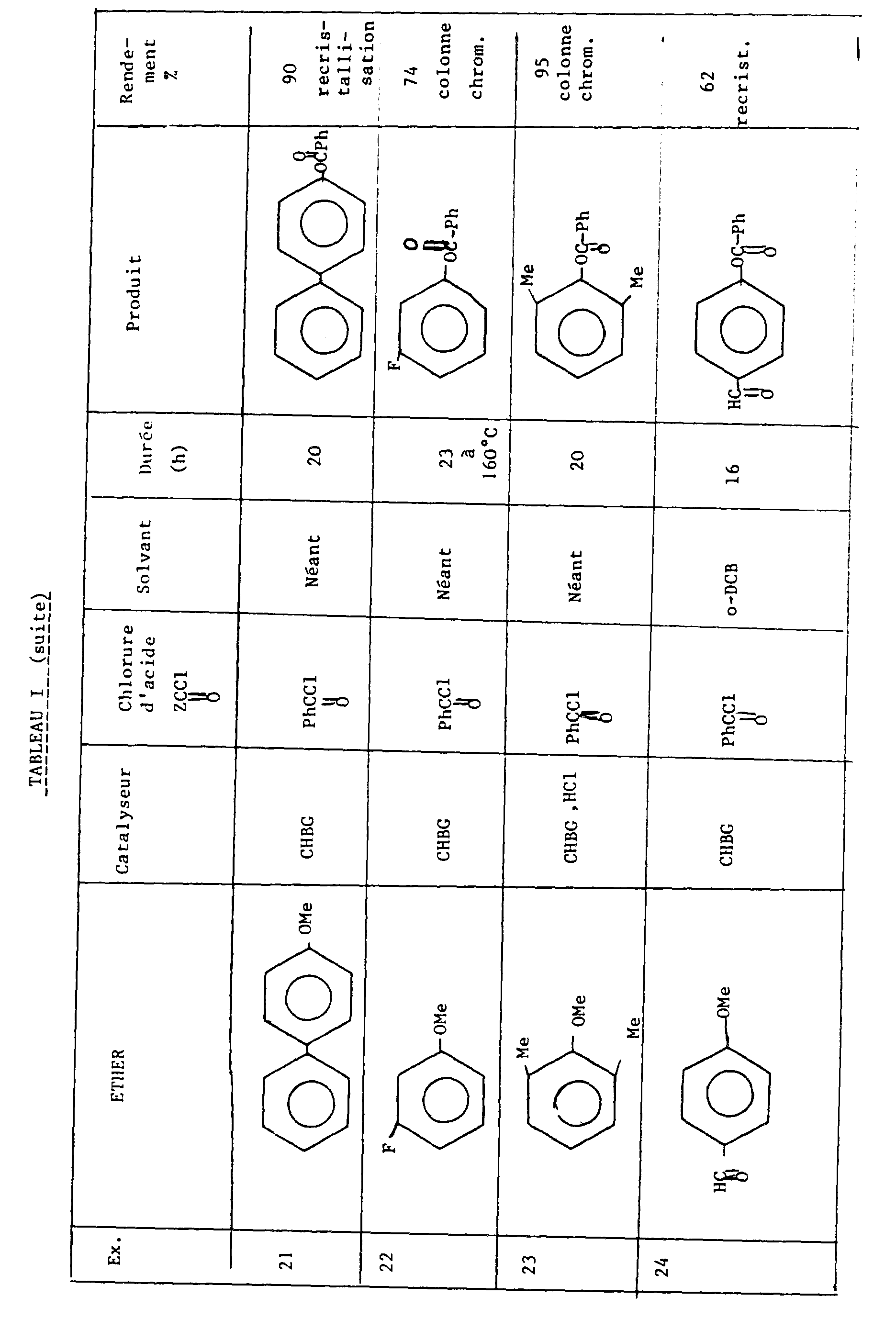

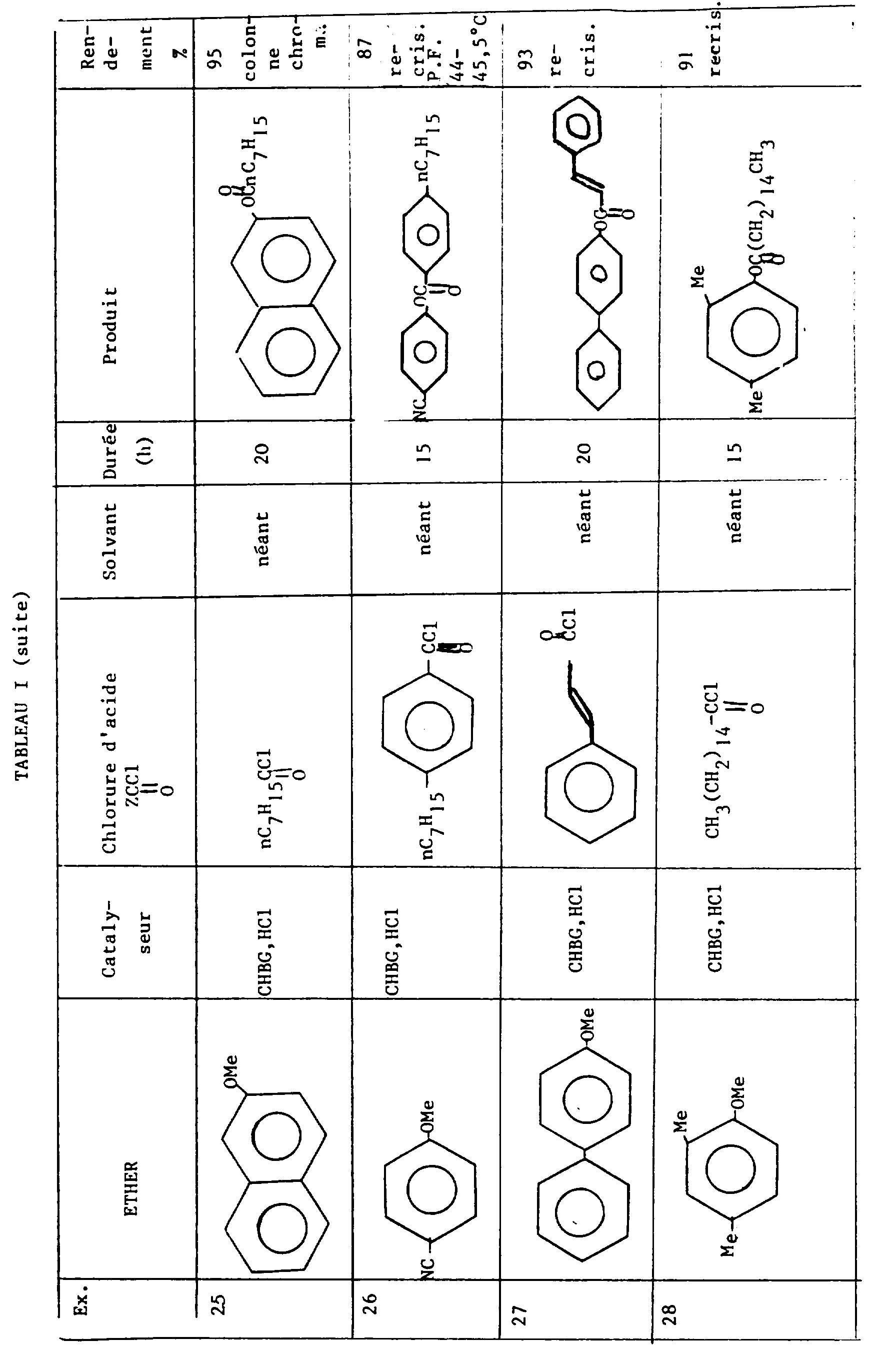

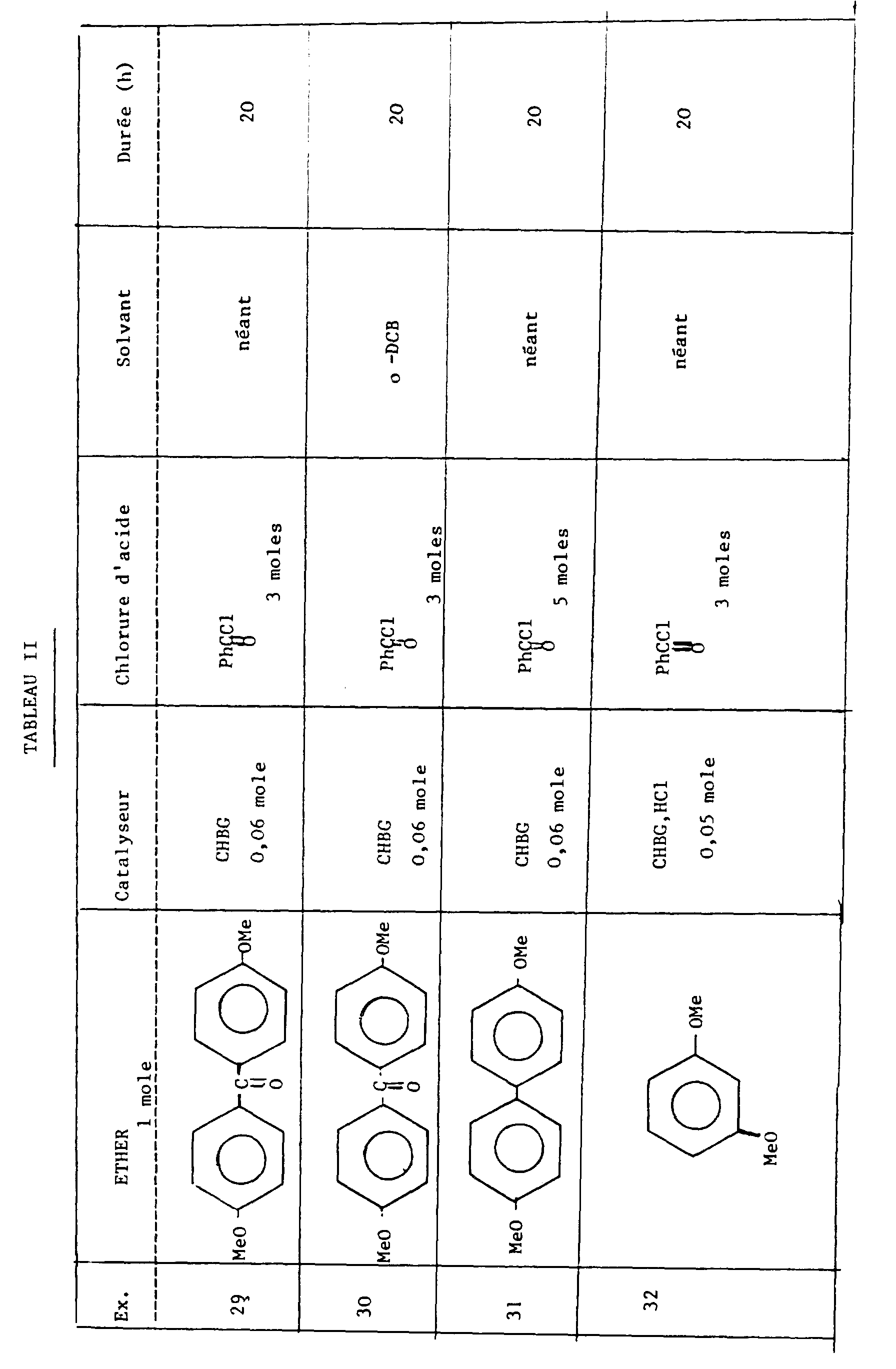

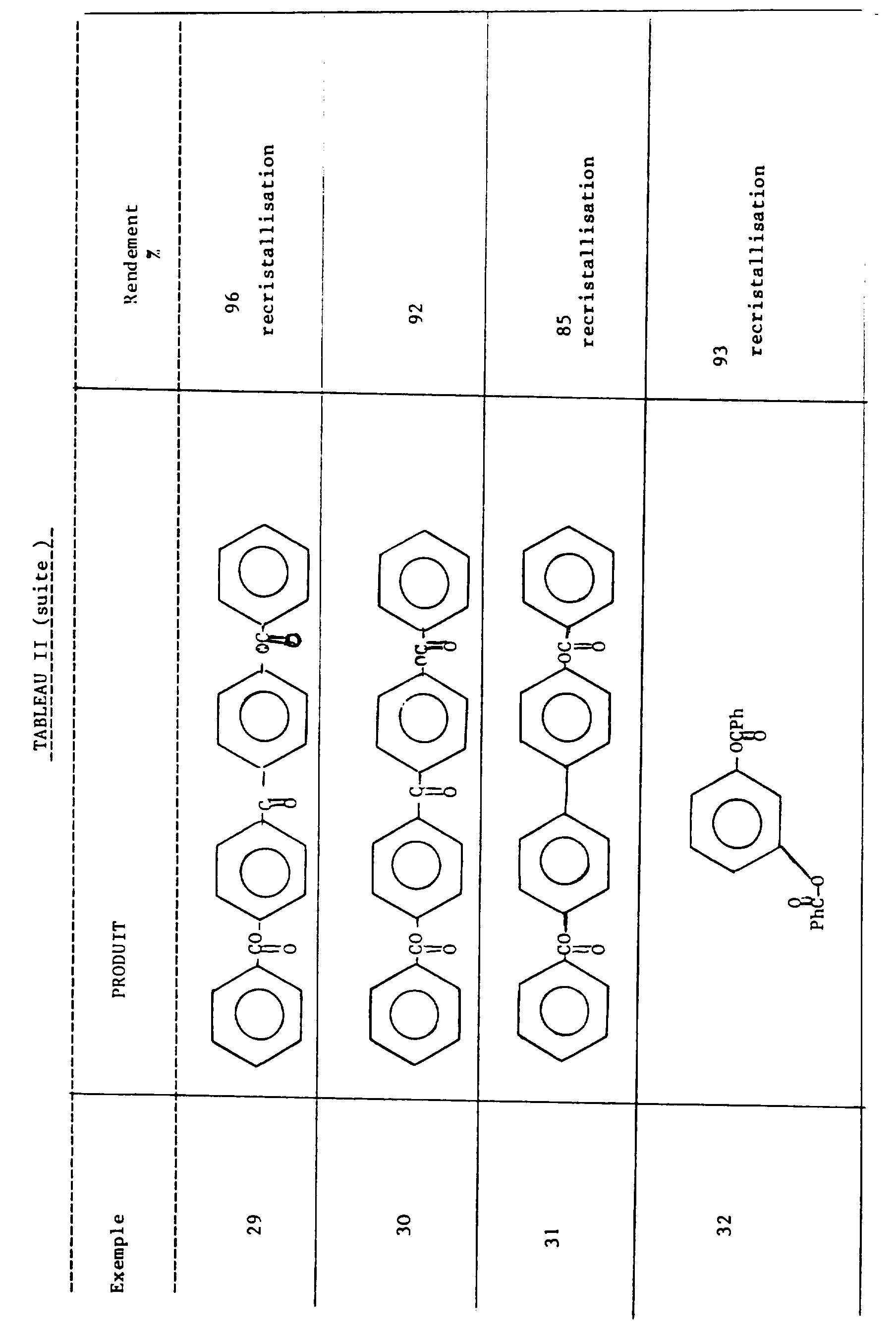

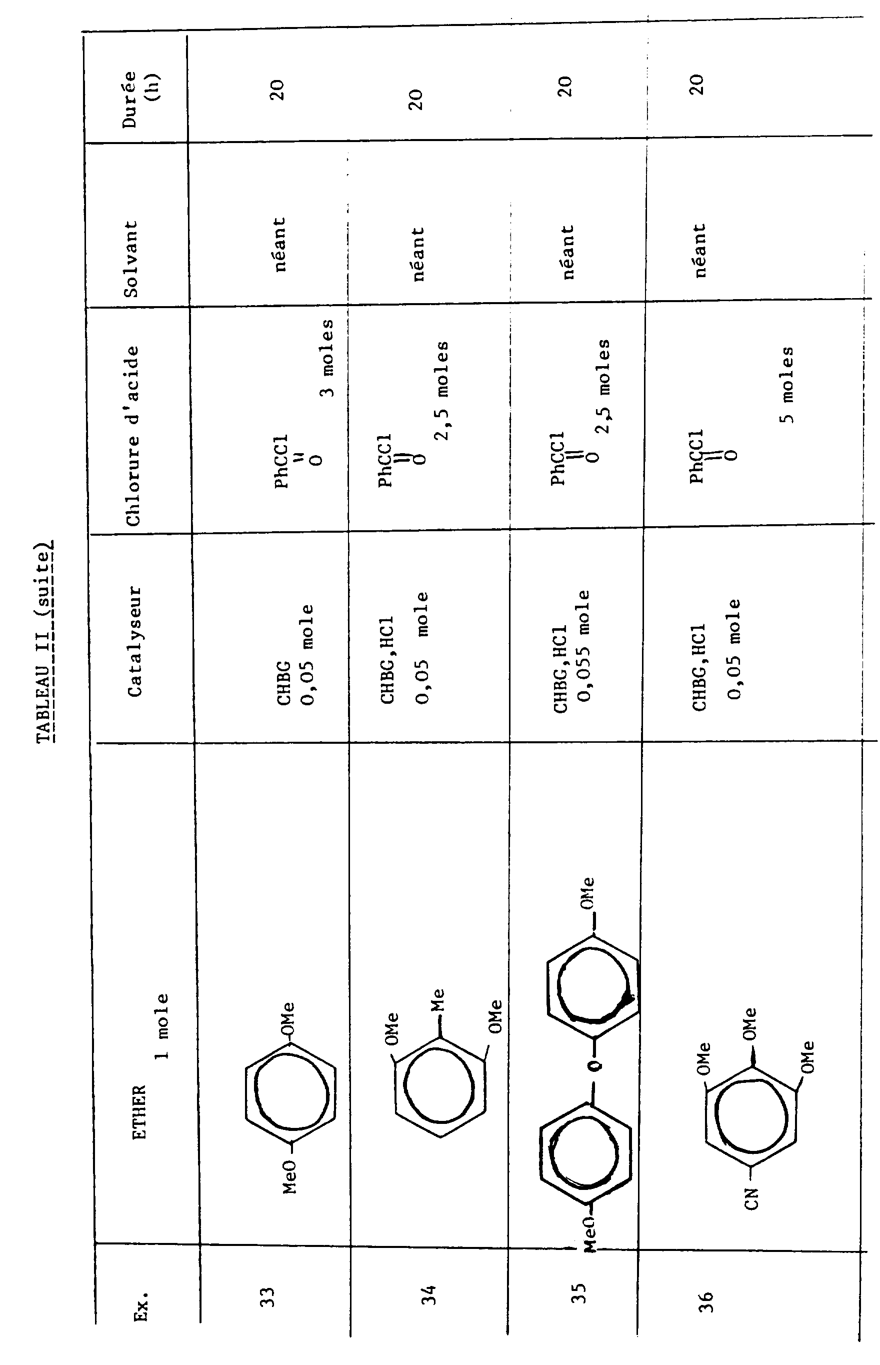

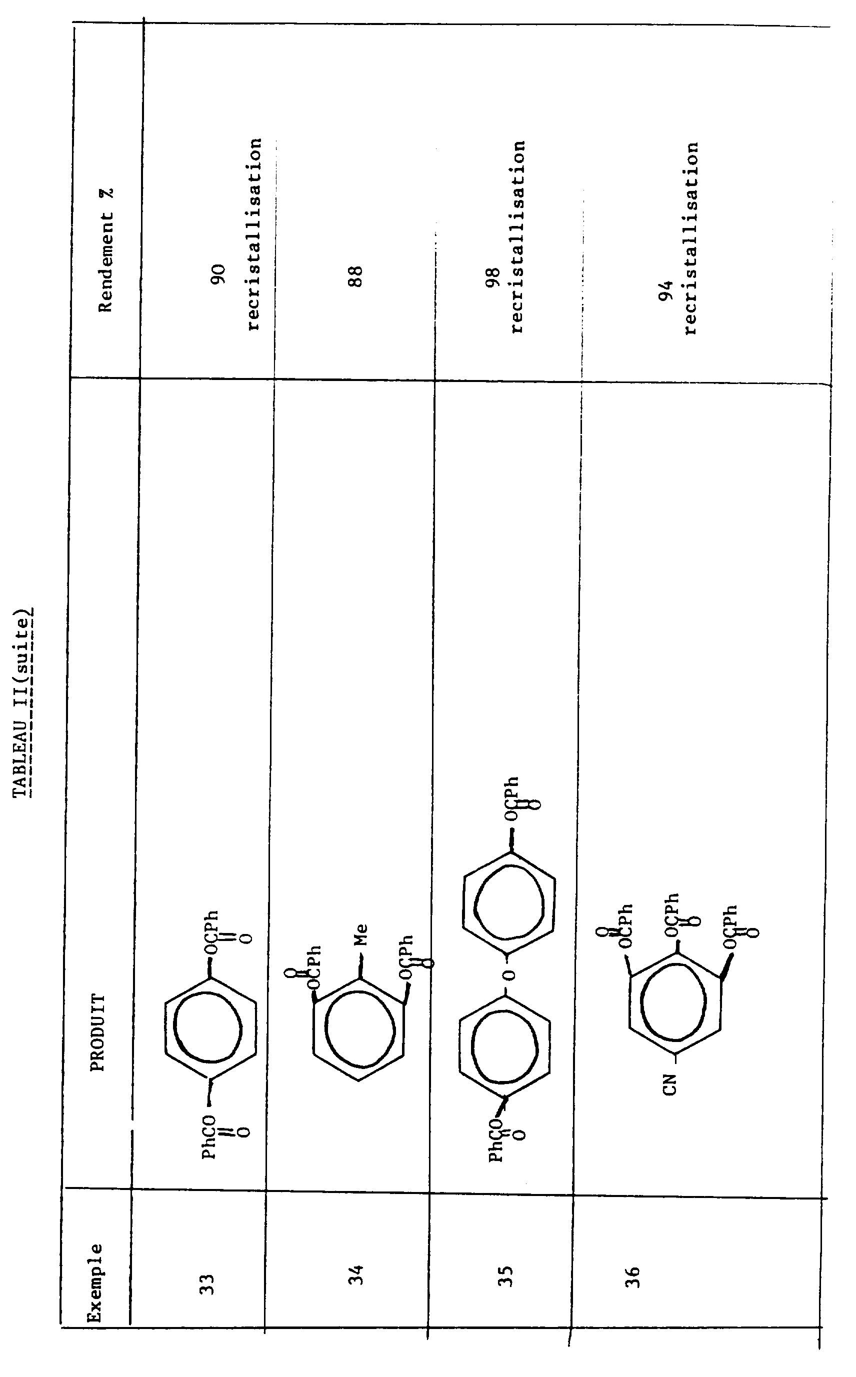

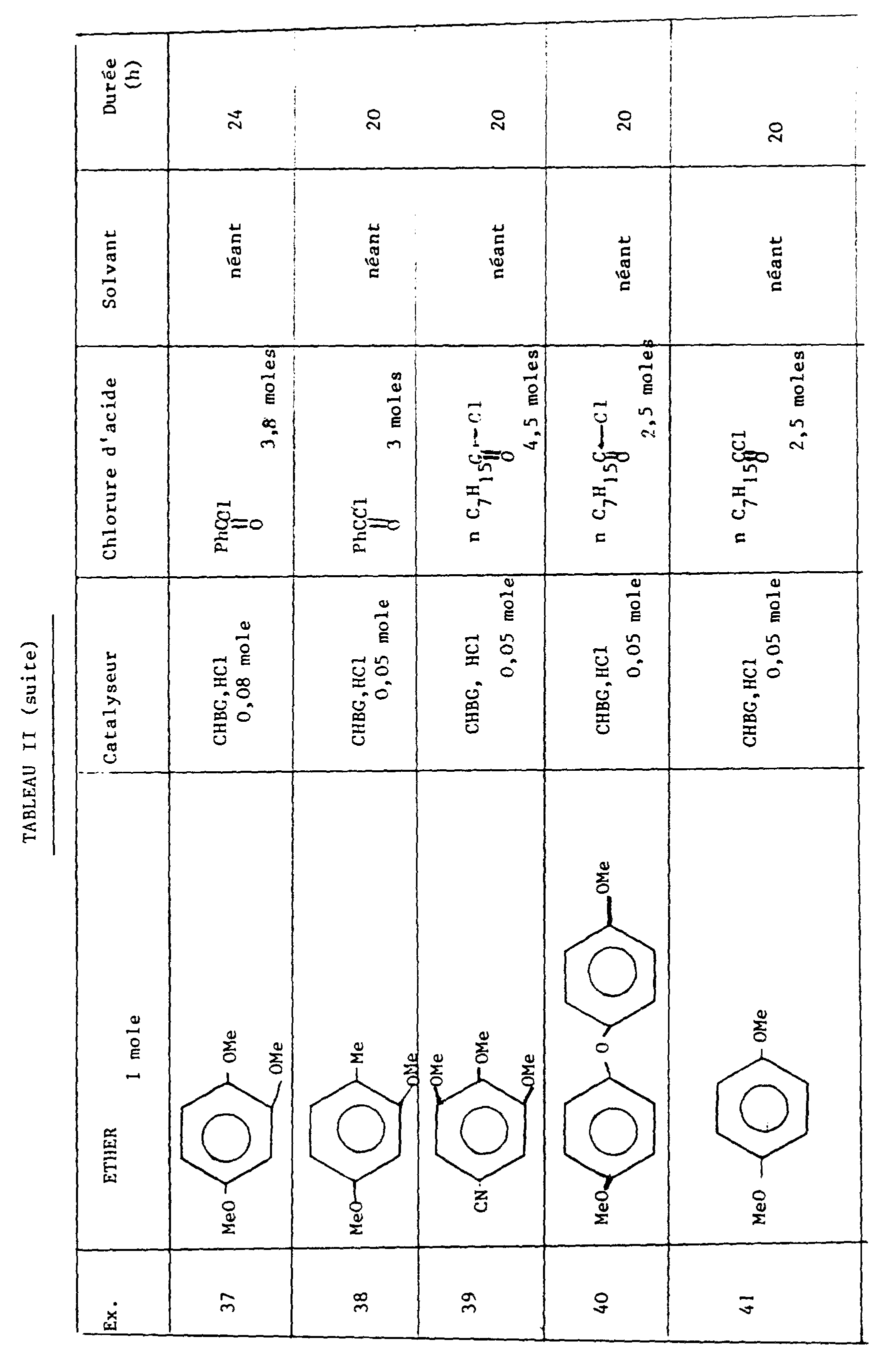

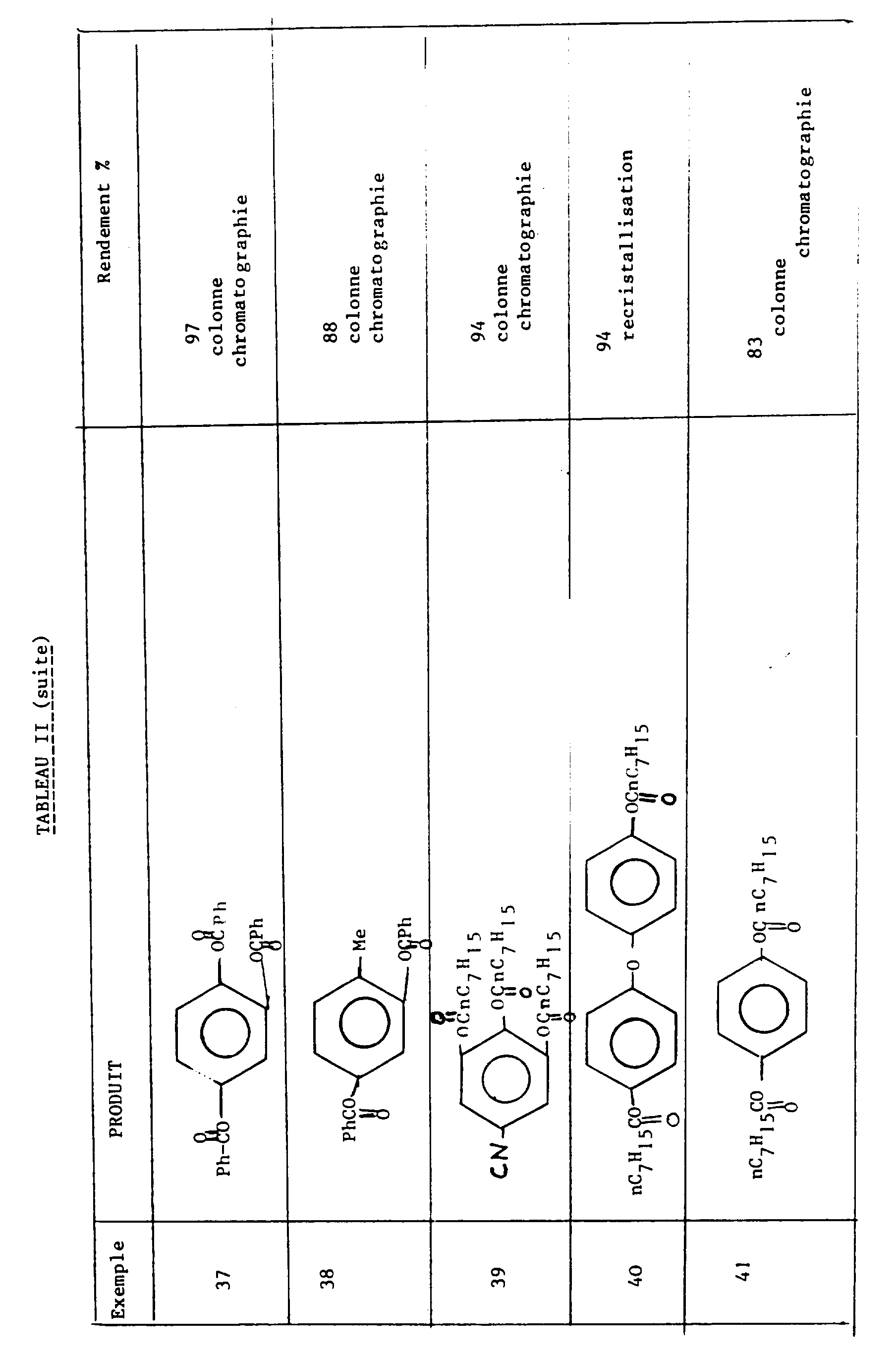

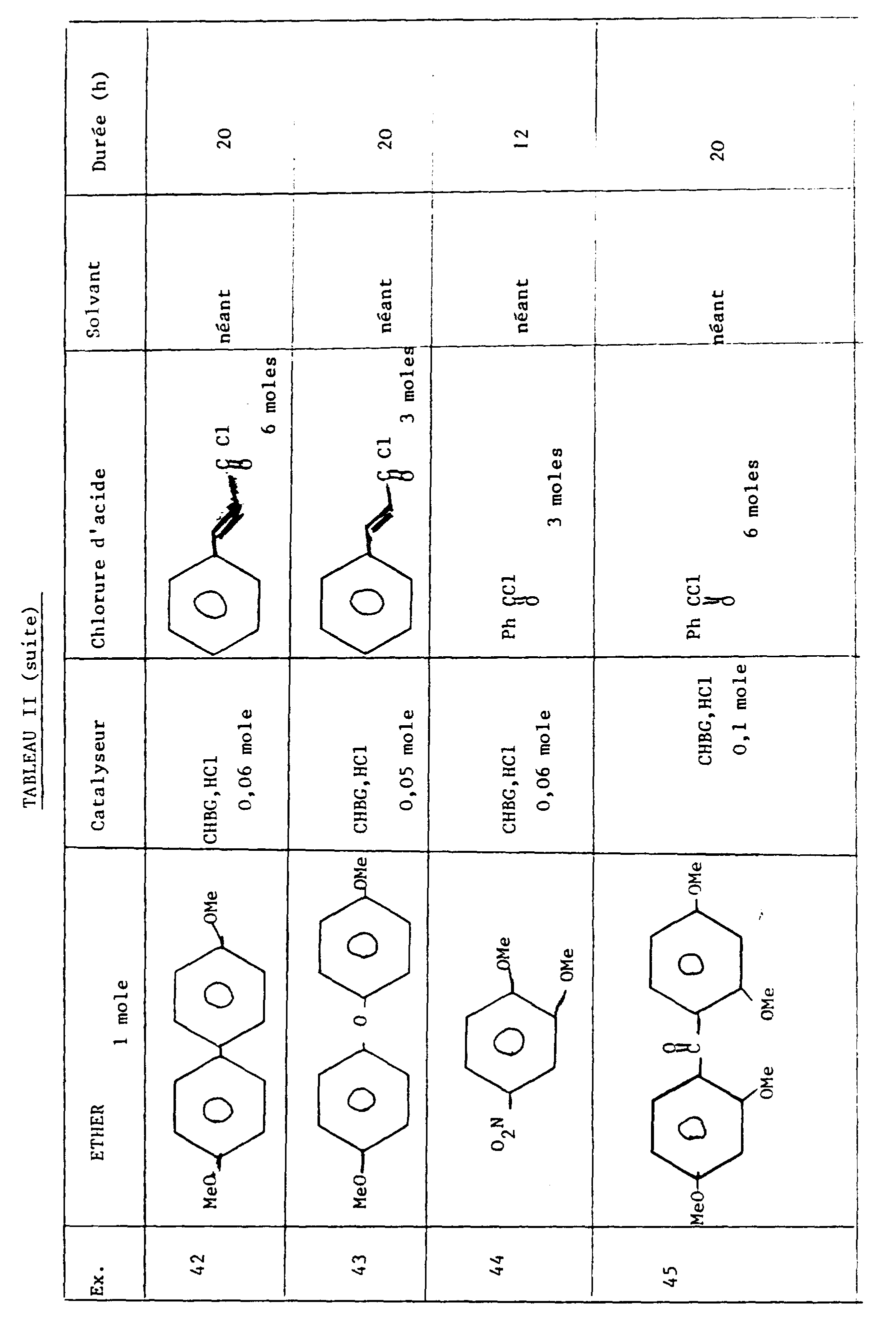

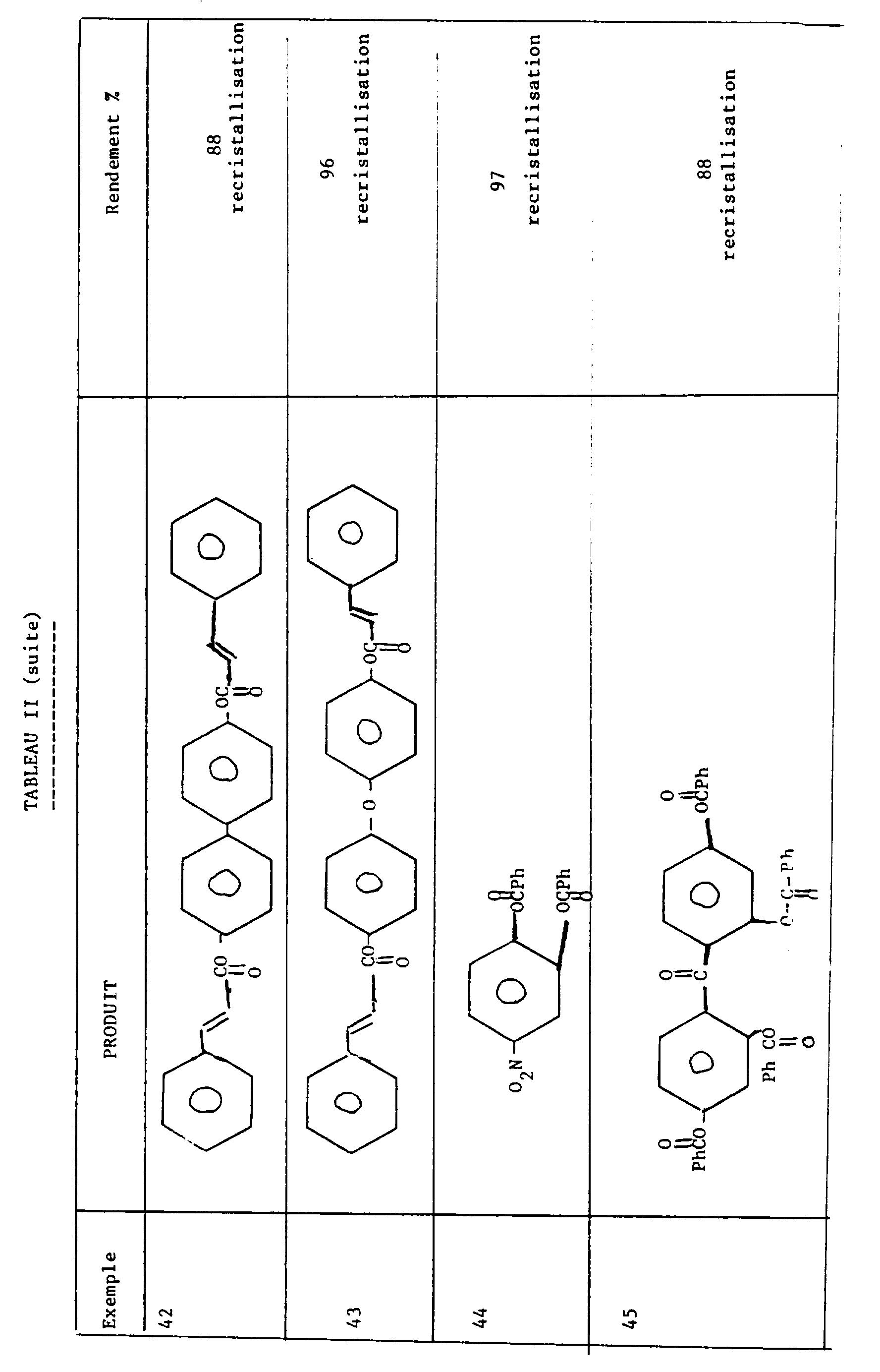

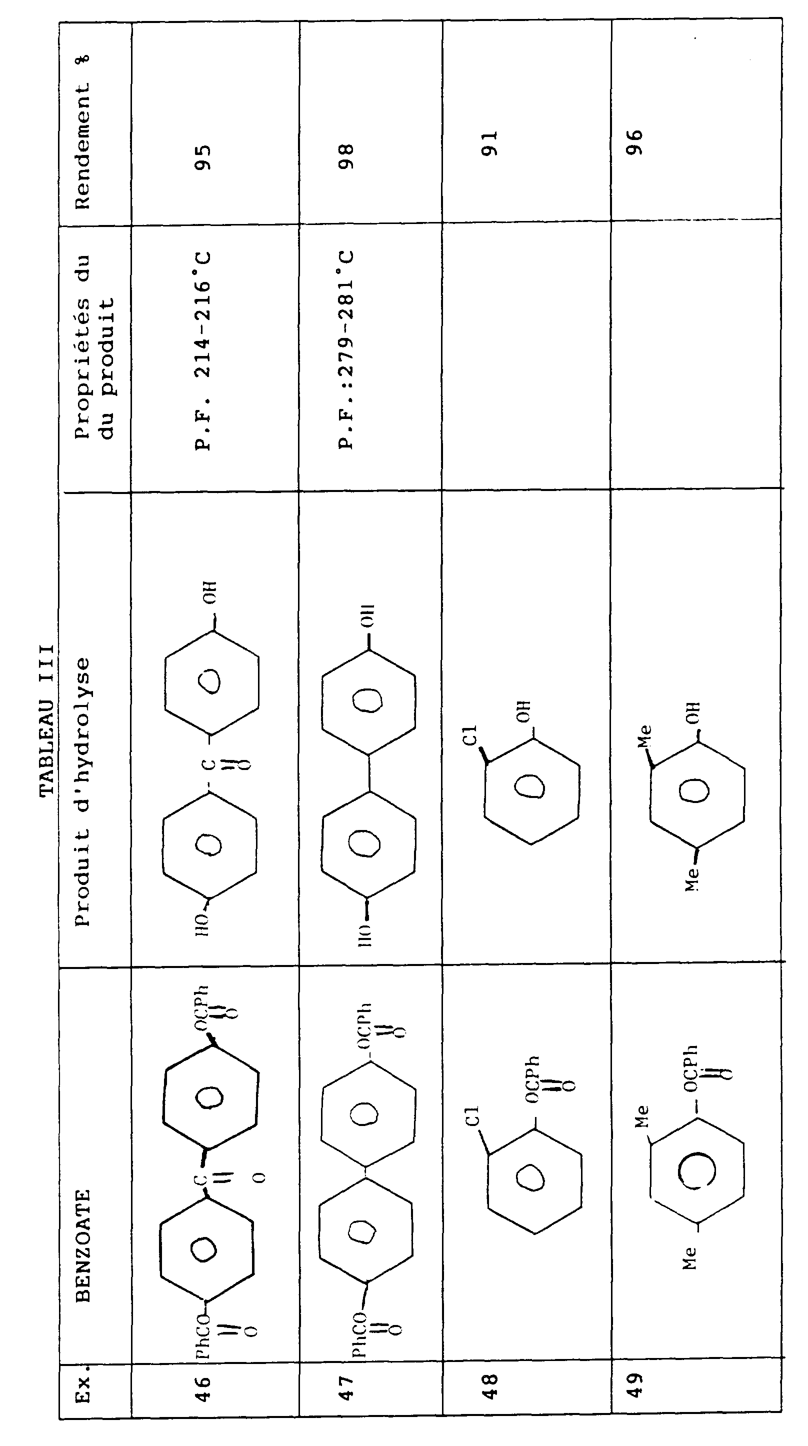

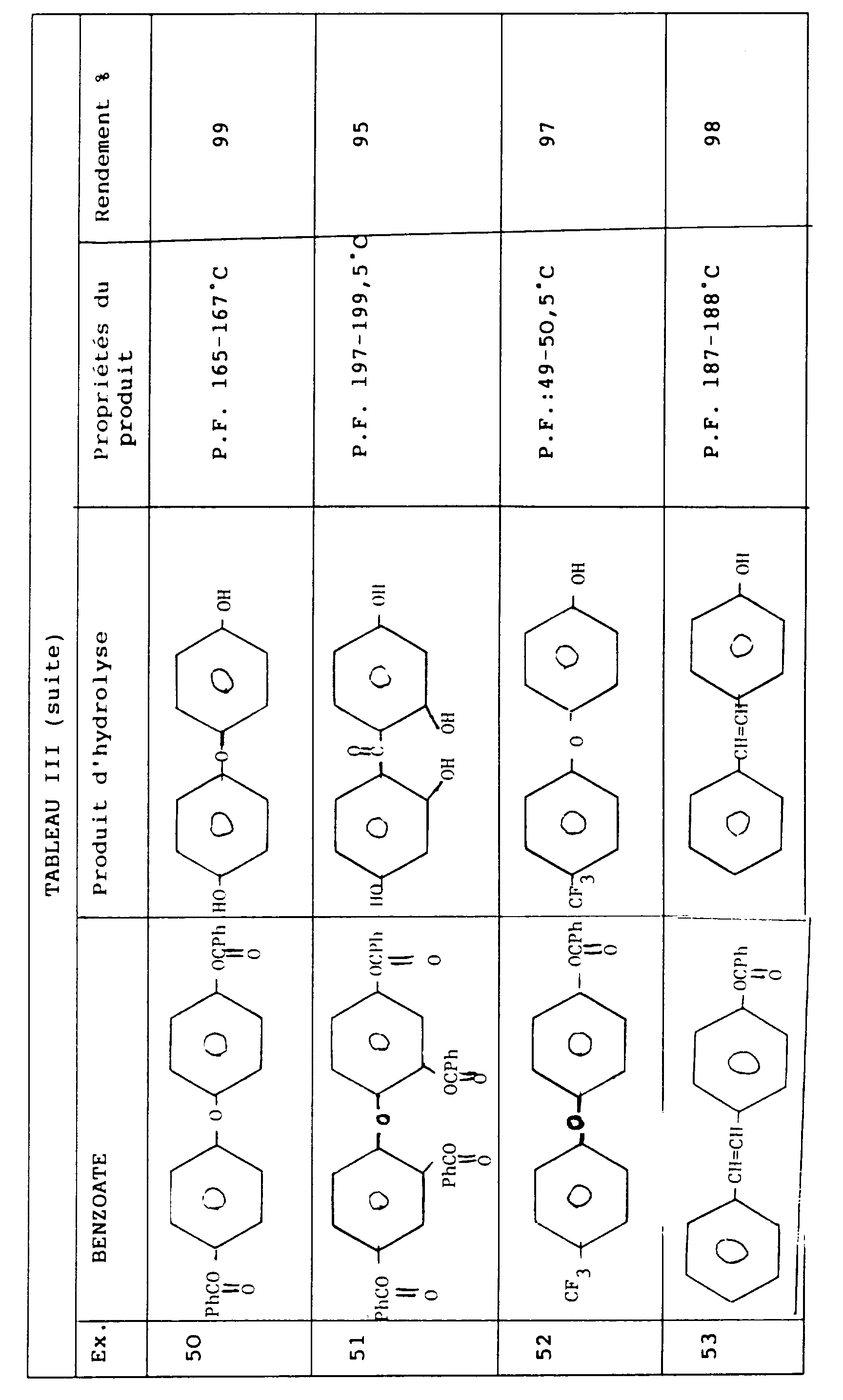

## Revendications

1. Procédé d'obtention d'un ester d'aryle par O-déalkylation d'un alkyl aryl éther, caractérisé en ce qu'on fait réagir un alkyl aryl éther avec un halogénure d'acyle en présence d'un catalyseur choisi parmi les sels d'hexaalkylguanidinium et les sels de tétraalkylphosphonium.

2. Procédé selon la revendication 1 , caractérisé en ce que les sels de guanidinium répondent à la formule : 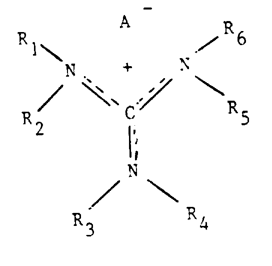 et les sels de phosphonium à la formule : 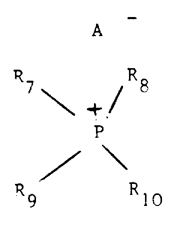 dans lesquelles R₁ à R₁₀ sont identiques ou différents et représentent des radicaux alkyles en C₁ à C₂₀ et A⁻ représente un anion choisi dans le groupe comprenant Cl⁻ Br⁻, I⁻, BF₄ ⁻, CN⁻, ClO₄ ⁻, NO₃ ⁻, NO₂ ⁻, OCN ⁻, CH₃SO₄ ⁻, HSO₄ ⁻.

3. Procédé selon l'une des revendications 1 et 2 , caractérisé en ce que le catalyseur est un chlorure d'hexaalkylguanidinium, utilisé en tant que tel ou sous forme de chlorhydrate .

4. Procédé selon la revendication 3 , caractérisé en ce que le catalyseur est le chlorure d'hexabutylguanidinium ou le chlorure d'hexaméthylguanidinium.

5. Procédé selon la revendication 2 , caractérisé en ce que les radicaux R₇ à R₁₀ contiennent de 4 à 18 atomes de carbone .

6. Procédé selon la revendication 5 , caractérisé en ce que le catalyseur est le bromure de tributylhexadécylphosphonium ou le bromure de tétrabutylphosphonium.

7. Procédé selon l'une des revendications 1 à 6 caractérisé en ce que l'alkyl aryl éther répond à la formule Ar(OR)ₙ dans laquelle :
R représente un groupement alkyle primaire ou secondaire en C₁ à C₆ , en particulier un groupement méthyle,
n est un nombre entier de 1 à 6 et Ar représente :
- un radical aromatique en C₆ à C₁₄ de préférence phényle ou naphtyle , éventuellement substitué par un ou plusieurs groupements identiques ou différents ,tels que des atomes d'halogène , en particulier F, Cl, Br, des groupements nitro , cyano, aldéhyde , des groupes alcoxy , aryloxy ou aryloxy substitué , par exemple des groupes alkylaryloxy ou halogénoalkylaryloxy, des groupes aryl- , alkyl- ou cycloalkylcétone, des groupes aryles en particulier phényle , des groupes alkyles en C₁ à C₂₀ , ramifié ou non, substitué ou non, par exemple par un groupe phényle ,
- le groupe coumarinyle ou
- un ensemble de plusieurs radicaux phényles, chaque radical phényle pouvant porter un ou plusieurs groupes OR.

8. Procédé selon l'une des revendications 1 à 7 , caractérisé en ce que l'halogénure d'acyle répond à la formule 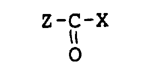 dans laquelle :
X représente un halogène , en particulier Br ou Cl,
Z représente
- un radical aromatique en C₆ à C₁₀, de préférence le radical phényle , éventuellement substitué par des radicaux choisis parmi les radicaux alkyle, les atomes d'halogène et les radicaux phénoxy,
- un radical aliphatique ou cycloaliphatique avec jusqu'à 20 atomes de carbone , de préférence avec de 6 à 20 atomes de carbone, saturé ou non , substitué ou non , par exemple par un groupe phényle,
- un radical phénoxy, lesdits radicaux pouvant également comporter ou être substitués par un autre groupe

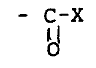

9. Procédé selon la revendication 7 , caractérisé en ce que Ar(OR)n est représenté par la formule générale : 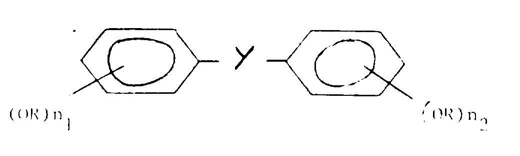 dans laquelle Y représente O, 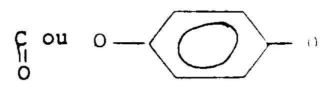 ou une liaison covalente , et
n₁, n₂ sont des nombres entiers de 1 à 3, identiques ou différents .

10. Procédé selon l'une quelconque des revendications 1 à 9 , caractérisé en ce que l'ester d'aryle obtenu est complémentairement hydrolysé en milieu basique.

11. Procédé d'obtention d'un polyester aromatique selon l'une des revendications 7 à 9 , caractérisé en ce que Z comporte un autre groupe 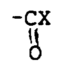 et n est un nombre entier supépérieur ou égal à 2 .

12. Procédé selon l'une des revendications 1 à 11 , caractérisé en ce qu'on emploie de 0,01à 0,1 équivalent de catalyseur par mole de fonction OR à cliver.

13. Procédé selon l'une des revendications 1 à 12, caractérisé en ce que la réaction est effectuée à une température comprise entre 150°C et 220°C et préférentiellement entre 180°C et 200°C.

14. Procédé selon l'une quelconque des revendications 1 à 13, caractérisé en ce que la réaction est effectuée en présence d'un solvant inerte.

## Patentansprüche

1. Verfahren zur Herstellung eines Arylesters durch O-Dealkylierung eines Alkylarylethers, dadurch gekennzeichnet, daß man einen Alkylarylether mit einem Acylhalogenid in Gegenwart eines Katalysators umsetzt, der aus den Hexaalkylguanidinium-Salzen und den Tetraalkylphosphonium-Salzen ausgewählt ist.

2. Verfahren gemäß Anspruch 1, dadurch gekennzeichnet, daß die Guanidinium-salze der Formel: 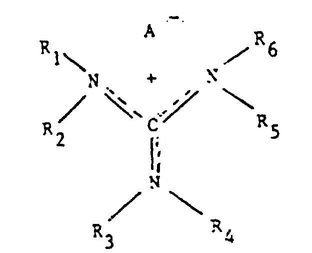 entsprechen, und die Phosphonium-Salze der Formel: 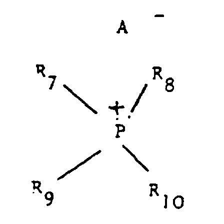 entsprechen, in denen R₁ bis R₁₀ identisch oder voneinander verschieden sind und Alyklreste von C₁ bis C₂₀ darstellen, und A⁻ ein Anion darstellt, das aus der Gruppe, bestehend aus Cl⁻, Br⁻, I⁻, BF₄ ⁻, CN⁻, ClO₄ ⁻, NO₃ ⁻, NO₂ ⁻,OCN⁻, CH₃SO₄ ⁻, HSO₄ ⁻, ausgewählt ist.

3. Verfahren gemäß einem der Ansprüche 1 und 2, dadurch gekennzeichnet, daß der Katalysator ein Hexaalkylguanidiniumchlorid ist, das als solches oder in Form des Chlorhydrats verwendet wird.

4. Verfahren gemäß Anspruch 3, dadurch gekennzeichnet, daß der Katalysator Hexabutylguanidiniumchlorid oder Hexamethylguanidiniumchlorid ist.

5. Verfahren gemäß Anspruch 2, dadurch gekennzeichnet, daß die Reste R₇ bis R₁₀ 4 bis 18 Kohlenstoffatome enthalten.

6. Verfahren gemäß Anspruch 5, dadurch gekennzeichnet, daß der Katalysator Tributylhexadecylphosphoniumbromid oder Tetrabutylhosphoniumbromid ist.

7. Verfahren gemäß einem der Ansprüche 1 bis 6, dadurch gekennzeichnet, daß der Alkylarylether der Formel Ar(OR)ₙ entspricht, worin:
R eine primäre oder sekundäre C₁-C₆-Alkylgruppe, insbesondere eine Methylgruppe ist;
n eine ganze Zahl von 1 bis 6 darstellt, und Ar darstellt:
- einen aromatischen C₆-C₁₄-Rest, vorzugsweise Phenyl oder Naphthyl, der gegebenenfalls durch eine oder mehrere identische oder verschiedene Gruppen, wie Halogenatome - insbesondere F, Cl, Br -, Nitro-, Cyano-, Aldehyd-, Alkoxy-, Aryloxy- oder substituierte Aryloxy-Gruppen, z.B. Alkylaryloxy- oder Halogenalkylaryloxy-Gruppen, Aryl-, Alkyl- oder Cycloalkylketon-Gruppen, Aryl-Gruppen, insbesondere Phenyl, gegebenenfalls verzweigte, gegebenenfalls substituierte C₁-C₂₀ Alkylgruppen, z.B. eine Phenylgruppe substituiert ist;
- die Coumarinyl-Gruppe, oder
- eine Gruppe verschiedener Phenylreste, wobei jeder Phenylrest eine oder mehrere OR-Gruppen umfassen kann.

8. Verfahren gemäß einem der Ansprüche 1 bis 7, dadurch gekennzeichnet, daß das Acylhalogenid der Formel 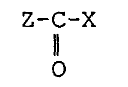 entspricht, worin
X ein Halogen, insbesondere Br oder Cl darstellt,
Z
- einen aromatischen C₆-C₁₀-Rest, vorzugsweise den Phenylrest darstellt, der gegebenenfalls durch Reste substituiert ist, die aus Alkylresten, Halogenatomen und Phenoxy-Resten ausgewählt sind,
- einen aliphatischen oder cycloaliphatischen Rest mit bis zu 20 Kohlenstoffatomen, vorzugsweise mit 6 bis 20 gegebenenfalls ungesättigten, gegebenenfalls substituierten Kohlenstoffatomen, z.B. eine Phenylgruppe, darstellt,
- einen Phenoxyrest darstellt, worin die genannten Reste gleichfalls eine andere 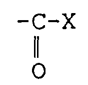 -Gruppe umfassen können , oder durch eine andere 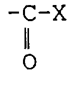 -Gruppe substituiert sein können.

9. Verfahren gemäß Anspruch 7, dadurch gekennzeichnet, daß Ar(OR)ₙ durch die allgemeine Formel 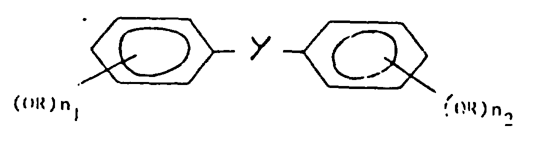 dargestellt ist, worin Y O, 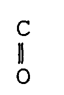 oder 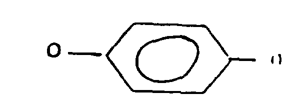 oder eine kovalente Bindung darstellt, und
n₁, n₂ identische oder voneinander verschiedene ganze Zahlen von 1 bis 3 sind.

10. Verfahren gemäß irgendeinem der Ansprüche 1 bis 9, dadurch gekennzeichnet, daß der erhaltene Arylester vollständig in basischem Medium hydrolysiert ist.

11. Verfahren zur Herstellung eines aromatischen Polyesters gemäß einem der Ansprüche 7 bis 9, dadurch gekennzeichnet, daß Z eine andere 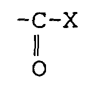 -Gruppe umfaßt, und
n eine ganze Zahl ist, die größer oder gleich 2 ist.

12. Verfahren gemäß einem der Ansprüche 1 bis 11, dadurch gekennzeichnet, daß man 0,01 bis 0,1 äquivalent Katalysator pro mol zu spaltender OR-Funktion verwendet.

13. Verfahren gemäß einem der Ansprüche 1 bis 12, dadurch gekennzeichnet, daß die Umsetzung bei einer Temperatur zwischen 150°C und 220°C, und vorzugsweise zwischen 180°C und 200°C, durchgeführt wird.

14. Verfahren gemäß irgendeinem der Ansprüche 1 bis 13, dadurch gekennzeichnet, daß die Umsetzung in Gegenwart eines inerten Lösungsmittels durchgeführt wird.

## Claims

1. Process for obtaining an aryl ester by O-dealkylation of an alkyl aryl ether, characterized in that an alkyl aryl ether is reacted with an acyl halide in the presence of a catalyst selected from hexaalkylguanidinium salts and tetraalkylphosphonium salts.

2. Process according to claim 1, characterized in that the guanidinium salts correspond to the formula: 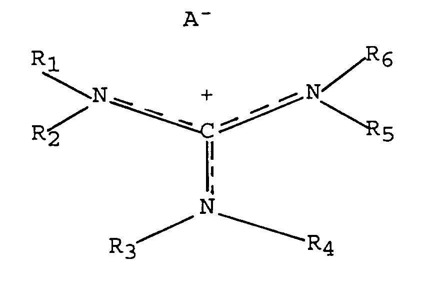 and the phosphonium salts to the formula: 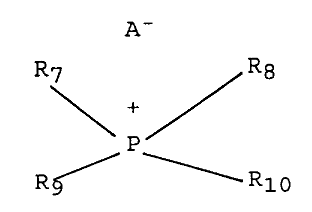 in which formulae R₁ to R₁₀ are identical or different and are C₁-C₂₀ alkyl groups and A⁻ is an anion selected from the group comprising Cl⁻, Br⁻, I⁻, BF₄ ⁻, CN⁻, C10₄ ⁻, NO₃ ⁻, NO₂ ⁻, OCN⁻, CH₃SO₄ ⁻ and HSO₄ ⁻.

3. Process according to one of claims 1 and 2, characterized in that the catalyst is a hexaalkylguanidinium chloride used as such or as an hydrochloride.

4. Process according to claim 3, characterized in that the catalyst is hexabutylguanidinium chloride or hexamethylguanidinium chloride.

5. Process according to claim 2, characterized in that the groups R₇ to R₁₀ have from 4 to 18 carbon atoms.

6. Process according to claim 5, characterized in that the catalyst is tributylhexadecylphosphonium bromide or tetrabutylphosphonium bromide.

7. Process according to one of claims 1 to 6, characterized in that the alkyl aryl ether corresponds to the formula Ar(OR)ₙ in which:
R is a primary or secondary C₁-C₆ alkyl group, in particular a methyl group,
n is an integer from 1 to 6 and Ar is:
- a C₆-C₁₄ aromatic group, preferably phenyl or naphthyl, optionally substituted by one or more, identical or different groups such as halogen atoms, in particular F, Cl, Br, nitro, cyano or aldehyde groups, alkoxy, aryloxy or substituted aryloxy groups, for example alkylaryloxy or haloalkylaryloxy groups, aryl-, alkyl- or cycloalkyl ketone groups, aryl groups, in particular phenyl, or C₁-C₂₀ alkyl groups, branched or unbranched, substituted or unsubstituted, for example by a phenyl group,
- the coumarinyl group or
- a group of a number of phenyl groups, it being possible for each phenyl group to carry one or more OR groups.

8. Process according to one of claims 1 to 7, characterized in that the acyl halide corresponds to the formula 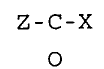
in which:
X is a halogen, in particular Br or Cl,
Z is
- a C₆-C₁₀ aromatic group, preferably the phenyl group, optionally substituted by groups selected from alkyl groups, halogen atoms and phenoxy groups,
- an aliphatic or cycloaliphatic group with up to 20 carbon atoms, preferably with 6 to 20 carbon atoms, saturated or unsaturated, substituted or unsubstituted, for example by a phenyl group,
- a phenoxy group,
it being possible for the said groups also to contain or be substituted by another 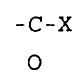 group.

9. Process according to claim 7, characterized in that Ar(OR)ₙ is denoted by the general formula: 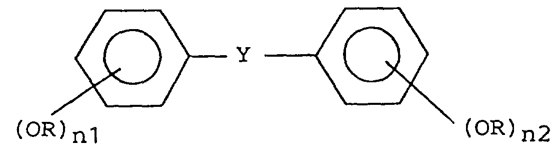 in which Y is O, 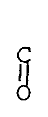 or O 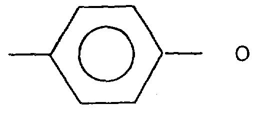 or a covalent bond, and
n₁ and n₂ are identical or different integers from 1 to 3.

10. Process according to any one of claims 1 to 9, characterized in that the aryl ester obtained is additionally hydrolyzed in basic medium.

11. Process for obtaining an aromatic polyester according to one of claims 7 to 9, characterized in that Z comprises another 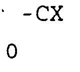 group and n is an integer greater than or equal to 2.

12. Process according to one of claims 1 to 11, characterized in that from 0.01 to 0.1 equivalent of catalyst is used per mole of OR functional group to be cleaved.

13. Process according to one of claims 1 to 12, characterized in that the reaction is performed at a temperature of between 150°C and 220°C and preferably between 180°C and 200°C.

14. Process according to any one of claims 1 to 13, characterized in that the reaction is performed in the presence of an inert solvent.
